# EUROPEAN PATENT SPECIFICATION

(11) **EP 2 718 425 B1**
(45) Date of publication and mention of the grant of the patent: **10.05.2017**
(21) Application number: 12726092.5
(22) Date of filing: 04.06.2012
(51) Int. Cl.: C12N 5/071, G01N 33/50

(54) **METHOD FOR DIFFERENTIATION OF PLURIPOTENT STEM CELLS INTO VASCULAR BED CELLS**
VERFAHREN ZUR DIFFERENZIERUNG PLURIPOTENTER STAMMZELLEN IN GEFÄSSBETTZELLEN
PROCÉDÉ DE DIFFÉRENCIATION DE CELLULES SOUCHES PLURIPOTENTES EN CELLULES DU LIT VASCULAIRE

(30) Priority: 09.06.2011 EP 11169316
(43) Date of publication of application: 16.04.2014
(73) Proprietor: F. Hoffmann-La Roche AG, 4070 Basel (CH)
(72) Inventor: CHRISTENSEN, Klaus, CH-4104 Oberwil BL (CH); GRAF, Martin, CH-4314 Zeiningen (CH); IACONE, Roberto, CH-4054 Basel (CH); PATSCH, Christoph, CH-4052 Basel (CH); THOMA, Eva, Christina, CH-4057 Basel (CH)
(74) Representative: Townsend, Carolin
(86) International application number: PCT/EP2012/060461
(87) International publication number: WO 2012/168167

(56) References cited:
- WO-A1-2010/099539
- TATSUMI RIE ET AL: "Simple and Highly Efficient Method for Production of Endothelial Cells From Human Embryonic Stem Cells", CELL TRANSPLANTATION, vol. 20, no. 9, 2011, pages 1423-1430, XP009162287, cited in the application
- JAMES DAYLON ET AL: "Expansion and maintenance of human embryonic stem cell-derived endothelial cells by TGF beta inhibition is Id1 dependent", NATURE BIOTECHNOLOGY, vol. 28, no. 2, February 2010 (2010-02), XP002682519, DOI: 10.1038/nbt.1605 cited in the application
- BAKRE M M ET AL: "Generation of multipotential mesendodermal progenitors from mouse embryonic stem cells via sustained Wnt pathway activation", JOURNAL OF BIOLOGICAL CHEMISTRY, AMERICAN SOCIETY FOR BIOCHEMISTRY AND MOLECULAR BIOLOGY, vol. 282, no. 43, 26 October 2007 (2007-10-26), pages 31703-31712, XP002561091, ISSN: 0021-9258, DOI: 10.1074/JBC.M704287200
- PURPURA K A ET AL: "Analysis of the temporal and concentration-dependent effects of BMP-4, VEGF, and TPO on development of embryonic stem cell-derived mesoderm and blood progenitors in a defined, serum-free media", EXPERIMENTAL HEMATOLOGY, ELSEVIER INC, US, vol. 36, no. 9, 1 September 2008 (2008-09-01), pages 1186-1198, XP025885166, ISSN: 0301-472X, DOI: 10.1016/J.EXPHEM.2008.04.003

## Description

This application relates to a method for differentiating pluripotent stem cells (PSCs) into vascular bed cells (i.e. endothelial cells (ECs) and/ or vascular smooth muscle cells (VSMCs)). Moreover this application relates to a method for differentiating human embryonic stem cells (ESCs) and induced pluripotent stem cells (iPSCs) into vascular bed cells based on linked steps of chemically defined medium inductions.

Almost all tissues of the human body depend on a blood supply. Endothelial cells form the linings of the blood vessels and provide a selective permeability barrier between the blood and tissues. In addition, vascular smooth muscle cells are found within the walls of blood vessels playing an important role in the control of vasoconstriction of the blood vessels. Endothelial cell injury, activation or dysfunction are major characteristics of the pathophysiology of many diseases.

Hence, an understanding of the fundamental principles of vascular biology is of utmost importance to discover novel approaches for treating many patients. For example, patients suffering from Diabetes Type-2 have a much higher incidence of coronary artery diseases (CAD) and peripheral vascular complications, like nephropathy and retinopathy. Thus, vascular complications are the major cause of the considerably increased mortality and morbidity rate in this patient population. Dysfunction of endothelial cells in the kidney leads to enhanced growth and vasoconstriction of vascular smooth muscle cells and mesangial cells resulting in a diffuse glomerulosclerosis and sequentially causing severe kidney failure. In diabetic retinopathy, hyperglycemia induces vascular smooth muscle cell death leading to a damage and increased growth of endothelial cells of the retinal capillaries results in a pathophysiological proliferation of the retina, which sequentially leads to impairment or even complete loss of the vision. Thus there is a need for patient specific in vitro vascular bed cell models to study vascular complications and to identify appropriate new targets and strategies to treat vascular complications in Diabetes Type-2 patients.

Endothelial cells are pivotal in angiogenesis and vasculogenesis, e.g. in response to pathological conditions such as chronic hypoxia or tissue ischemia. Growth factors, such as the vascular endothelial growth factor (VEGF), can activate the endothelial signaling cascade resulting in cell proliferation and new tubes formation. Endothelial progenitors cells (EPC) are recently under focus as potential therapeutic treatment in vascular regenerative medicine: Effective neovascularization induced by EPC transplantation for hindlimb, myocardial and cerebral ischemia has been assessed in many preclinical studies, as well as in clinical trials for the treatment of ischemic cardiovascular diseases in chronic and acute coronary artery diseases (Yamahara K, Itoh H., Ther Adv Cardiovasc Dis. 2009 Feb;3(1):17-27; Kawamoto A, Asahara T., Catheter Cardiovasc Interv. 2007 Oct 1;70(4):477-84; Marsboom G, Janssens S., Expert Rev Cardiovasc Ther. 2008 Jun;6(5):687-701.). One of the major drawbacks to the potential application of autologous ECs/EPCs in cell-based therapy is their limited expansion ability and availability.

Drug-induced toxicity frequently causes injury of principal organs such as the liver and the lung. The toxic drug or its metabolite affect the biology of target cells directly or provoke an immune response within the organ. When liver sinusoidal endothelial cells and pulmonary endothelial cells are affected by drug-induced toxicity, severe tissue edema develops, which can lead to hepatic failure or dyspnea. The development of patient specific in vitro endothelial cell models would allow assessing the in vitro drug toxicity and facilitating the development of drugs with a decreased risk of causing tissue edema.

Embryonic stem (ES) cells and patient specific induced pluripotent stem cells (iPSCs) are a potential source for the production of endothelial cells and endothelial progenitor cells in large scale for regenerative vascular medicine. With the induced pluripotent stem cells (iPSCs) technology (Takahashi, K. & Yamanaka, S.,"Induction of pluripotent stem cells from mouse embryonic and adult fibroblast cultures by defined factors", Cell 126, 663-676 (2006)) somatic cells can be reprogrammed to iPSCs by transduction of four defined factors (Sox2, Oct4, Klf4, c-Myc). The iPSC technology enables the generation of patient specific iPSCs, which can be differentiated into patient specific endothelial cells. These patient specific endothelial cells are useful for example in *in vitro* modeling of the pathophysiology of vascular complications associated with Diabetes Type-2, or for the assessment of drug toxicity.

One important prerequisite to attempt such in vitro disease modeling is the implementation of an efficient, robust and scalable differentiation system (Grskovic et al., 2011; Tiscornia et al., 2011; Zhu et al., 2011). Previous efforts to differentiate human PSCs into vascular bed cells have not achieved scales and efficacies relevant for drug discovery campaigns or regenerative cell therapies. Several methods for differentiating human pluripotent stem cells (embryonic stem cells and iPSCs) into endothelial cells have been published. Endothelial cells derived from pluripotent stem cells are defined by the minimal requirement of expressing surface markers, such as CD31 and CD 144, and by the ability to form tubes in matrigel coated plates. James et al. (Nature Biotech 28, 161-167, 2010) differentiated endothelial cells from embryoid body cultures derived from embryonic stem cells. Therein, the embryoid bodies were sequentially stimulated with different growth factors like bone morphogenetic protein 4, activin A, fibroblast growth factor 2 and VEGF-A. The authors used a TGFb inhibitor small molecule (e.g. SB431542) in order to increase the yield and the reproducibility of the endothelial cell differentiation. However, this method has major drawbacks: Firstly, the absolute yield of endothelial cells was only below 10% and secondly, the overall time needed to differentiate pluripotent stem cells into endothelial cell was 10 days at the minimum. Recently, Tatsumi et al. (Cell Transplantation, 2010) have circumvented some of these limitations with a new approach for the differentiation of human embryonic stem cell-derived endothelial cells. Using a differentiation method consisting of a stepwise combination of 3 days incubation in a chemically defined serum free medium supplemented with 5 µM of a glycogen synthase kinase-3b (GSK-3b) inhibitor ((2'Z, 3'E)-6-bromoindirubin-3'-oxime, referred to as "BIO") and 2 days incubation in vascular endothelial growth factor (VEGF)-supplemented medium, endothelial cells are induced in 5 days with about 20% efficiency determined by endothelial cells expressing the surface marker CD 144 (further depicted as CD 144+ cells). The CD 144+ endothelial cells are then sorted using a magnetic-activated cell sorting (MACS) separation and expanded for different passages in VEGF supplemented medium. Thereafter, the differentiated endothelial cells have been tested for some of the hallmark characteristics of endothelial cells: capillary-like tube formation, uptake of 1',1'-dioctadecyl-3,3,3', 3'-tetramethylindocarbocyanine-labeled acetylated low-density lipoprotein (DiI-Ac-LDL), and the expression of markers CD31, CD34, CD 144 and VEGFR-2. With this method the overall time needed for differentiation of the endothelial cells was only 5 days, however the absolute yield (about 20 %) of endothelial cells was only slightly increased. This approach has additional drawbacks: In the initial step, undifferentiated human embryonic stem cells are dissociated into small cell clumps and plated onto type I collagen-coated dishes. With the use of small embryonic stem cell clumps, the starting conditions are poorly defined, which has a negative impact on reproducibility.

In summary, the major drawback of all known differentiation methods is the requirement of undefined factors such as serum, conditioned medium, co-cultures with mouse OP9 stromal cells or feeder layers, the heterogeneous nature of cells aggregates or embryoid bodies (James et al., 2010, Sumi et al., 2008; Vodyanik et al., 2005; Wang et al., 2007; Levenberg et al., 2002 PNAS; Kane et al., 2010; Tatsumi et al., 2010).

There remains a need for an easy accessible and reproducible method for the differentiation of pluripotent stem cells into vascular bed cells. The present invention provides an improved method for differentiating pluripotent stem cells into endothelial cells in a decreased amount of time (5 days) and with a significantly increased yield (up to 85%, as determined by cells expressing marker CD144), and can be reproduced entirely. The new method alleviates the necessity of obtaining embryoid bodies or small cell clumps from pluripotent stem cells and removes the major drawback of low reproducibility and standardization of methods known in the art. Moreover, the high efficiency (up to 85% yield of endothelial cells expressing marker CD 144) makes it now possible to use these endothelial cells in large scales in drug discovery and safety, in regenerative medicine applications, and in *in vitro* disease modeling in the pharmaceutical industry. In addition, the new method allows for selective modulation of the vascular progenitor cells, which enables shifting lineage commitment either predominantly into endothelial (∼85%) or into vascular smooth muscle cells (∼90%).

Provided herein is a method for differentiating pluripotent stem cells into vascular bed cells, said method comprising the steps of:
a) providing a monolayer of pluripotent stem cells in a pluripotency medium
b) incubating said cells in a priming medium supplemented with a small molecule that activates the Beta-Catenin (cadherin-associated protein, beta 1; human gene name CTNNB1) pathway and/or the Wnt receptor signaling pathway and/or hedgehog (HH) signaling pathway, wherein the small molecule is 3-(3-Amino-phenyl)-4-(1-methyl-1 H-indol-3-yl)-pyrrole-2,5-dione
c) inducing the differentiation by incubating said primed cells in an induction medium.

Preferably the media are changed in between each steps, that means that the first medium is discarded e.g. by aspiration before the second medium is added to the cells.

"A monolayer of pluripotent cells" as used herein means that the pluripotent stem cells are provided in single cells which are attached to the adhesive substrate in one single film, as opposed to culturing cell clumps or embryoid bodies in which a solid mass of cells in multiple layers form various three dimensional formations attached to the adhesive substrate.

Providing a monolayer of pluripotent stem cells in the initial step is crucial for the reproducibility and efficiency of the method and has not been described before in any methods for differentiation of vascular bed cells. In one embodiment, monolayers of pluripotent stem cells can be produced by enzymatically dissociating the cells into single cells and bringing them onto an adhesive substrate, such as pre-coated matrigel plates (e.g. BD Matrigel hESC-qualified from BD Bioscience, Geltrex hESC-qualified from Invitrogen, Synthemax from Corning). Examples of enzymes suitable for the dissociation into single cells include Accutase (Invitrogen), Trypsin (Invitrogen), TrypLe Express (Invitrogen). In one embodiment, 20000 to 60000 cells per cm² are plated on the adhesive substrate. The medium used herein is a pluripotency medium which facilitates the attachment and growth of the pluripotent stem cells as single cells in a monolayer. "Pluripotency medium" as used herein refers to any chemically defined medium useful for the attachment of the pluripotent stem cells as single cells on a monolayer while maintaining their pluripotency and are well known in the art. The "pluripotency medium" refers to a medium that contains at least one of the following growth factors: basic fibroblast growth factor (bFGF, also depicted as Fibroblast Growth Factor 2, FGF2) and transforming growth factor β (TGFβ). In one embodiment, the pluripotency medium is a serum free medium supplemented with a small molecule inhibitor of the Rho-associated coiled-coil forming protein serine/threonine kinase (ROCK) family of protein kinases (herein referred to as ROCK kinase inhibitor).

Thus, in one embodiment, step a) of the method described above comprises providing a monolayer of pluripotent stem cells in a pluripotency medium, wherein said pluripotency medium is a serum free medium supplemented with a ROCK kinase inhibitor.

Examples of serum-free media suitable for the attachment are mTeSR1 or TeSR2 from Stem Cell Technologies, Primate ES/iPS cell medium from ReproCELL and StemPro hESC SFM from Invitrogen, X-VIVO from Lonza. Examples of ROCK kinase inhibitor useful herein are Fasudil (1-(5-Isoquinolinesulfonyl)homopiperazine), Thiazovivin (N-Benzyl-2-(pyrimidin-4-ylamino)thiazole-4-carboxamide) and Y27632 ((+)-(*R*)-*trans*-4-(1-aminoethyl)-*N*-(4-pyridyl) cyclo-hexanecarboxamide dihydrochloride, e.g. Catalogue Number: 1254 from Tocris bioscience). In one embodiment, the pluripotency medium is a serum free medium supplemented with 2-20 µM Y27632, preferably 5-10 µM Y27632. In another embodiment the pluripotency medium is a serum free medium supplemented with 2-20 µM Fasudil. In another embodiment the pluripotency medium is a serum free medium supplemented with 0.2-10 µM Thiazovivin.

In one embodiment step a) of the method described above comprises providing a monolayer of pluripotent stem cells in a pluripotency medium and growing said monolayer in the pluripotency medium for one day (24 hours). In another embodiment step a) of the method described above comprises providing a monolayer of pluripotent stem cells in a pluripotency medium and growing said monolayer in the pluripotency medium for 18 hours to 30 hours, preferably for 23 to 25 hours.

In another embodiment step a) of the method described above comprises providing a monolayer of pluripotent stem cells in a pluripotency medium, wherein said pluripotency medium is a serum-free medium supplemented with a ROCK kinase inhibitor, and growing said monolayer in the pluripotency medium for one day (24 hours). In another embodiment step a) of the method described above comprises providing a monolayer of pluripotent stem cells in a pluripotency medium, wherein said pluripotency medium is a serum-free medium supplemented with a ROCK kinase inhibitor, and growing said monolayer in the pluripotency medium for 18 hours to 30 hours, preferably for 23 to 25 hours.

A "suitable medium for priming", also depicted as "priming medium", as used herein refers to any chemically defined medium useful for priming of the pluripotent stem cells towards endothelial cells. As used herein, "priming medium" refers to a medium that contains at least one factor, such as a small molecule that activates the Beta-Catenin (cadherin-associated protein, beta 1; human gene name CTNNB1) pathway and/or the Wnt receptor signaling pathway and/or hedgehog (HH) signaling pathway, that promotes the induction activity of mesoderm. Upon incubation in priming medium, the pluripotent stem cells start to change cell morphology overtime and the cell proliferation is increased. The "priming" step is defined by the expression of specific genes and markers involved into the mesoderm transition (e.g. GATA2, VIMENTIN, SMA, HAND1, FOXa2 (low expression), KDR) and down regulation of the pluripotency associated genes and markers (e.g. OCT4 (POU5F1), NANOG, SOX2, REX1 (ZFP42), LEFTY1, LEFTY2, TDGF1, DNMT3B, GABRB3, GDF3, TERT).

Said small molecules activating Beta-Catenin (cadherin-associated protein, beta 1; human gene name CTNNB1) pathway and/or the Wnt receptor signaling pathway and/or hedgehog (HH) signaling pathway is a small molecule inhibitor of glycogen synthase kinase 3 (Gsk3a-b). The disclosure also relates to small molecule inhibitors of CDC-like kinase 1 (Clkl-2-4), small molecule inhibitors of mitogen-activated protein kinase 15 (Mapk15), small molecule inhibitors of dual-specificity tyrosine-(Y)-phosphorylation regulated kinase (Dyrkla-b 4), small molecule inhibitors of cyclin-dependent kinase 16 (Pctk1-3 4), Smoothened (SMO) activators and modulators of the interaction between β-catenin (or γ-catenin) and the coactivator proteins CBP (CREB binding protein) and p300 (E1A binding protein p300).

Said glycogen synthase kinase 3 (Gsk3a-b) inhibitor is a pyrrolidindione-based GSK3 inhibitor. "Pyrrolidindione-based GSK3 inhibitor" as used herein relates to selective cell permeable ATP-competitive inhibitors of GSK3α and GSK3β with low IC₅₀ values.

Said pyrrolidindione-based GSK3 inhibitor is (3-(3-Amino-phenyl)-4-(1-methyl-1H-indol-3-yl)-pyrrole-2,5-dione (CP21R7, also referred to as "compound 21" herein; see e.g. L. Gong et al; Bioorganic& Medicinal Chemistry Letters 20 (2010), 1693-1696). The disclosure also relates to said pyrrolidindione-based GSK3 inhibitor selected from the group comprising 3-(2,4-Dichlorophenyl)-4-(1-methyl-1H-indol-3-yl)-1H-pyrrole-2,5-dione (SB216763), 3-[(3-Chloro-4-hydroxyphenyl)amino]-4-(2-nitrophenyl)-1H-pyrrol-2,5-dione (SB415286), *N*⁶-{2-[4-(2,4-Dichloro-phenyl)-5-imidazol-1-yl-pyrimidin-2-ylamino]-ethyl}-3-nitro-pyridine-2,6-diamine 2HCl, 3-Imidazo[1,2-*a*]pyridin-3-yl-4-[2-(morpholine-4-carbonyl)-1,2,3,4-tetrahydro-[1,4]diazepino[6,7,1-*hi*]indol-7-yl]-pyrrole-2,5-dione, 9-Bromo-7,12-dihydro-indolo[3,2-d]-[1]benzazepin-6(5H)-one (Kenpaullone), and 9-Bromo-7,12-dihydro-pyrido[3',2':2,3]azepino[4,5-b]indol-6(5H)-one (CHIR99021).

The disclosure also relates to said CDC-like kinase 1 (Clkl-2-4) inhibitor selected from the group comprising benzothiazole and 3-Fluoro-*N*-[1-isopropyl-6-(1-methyl-piperidin-4-yloxy)-1,3-dihydro-benzoimidazol-(2E)-ylidene]-5-(4-methyl-1*H*-pyrazole-3-sulfonyl)-benzamide.

The disclosure also relates to said mitogen-activated protein kinase 15 (Mapk15) inhibitor selected from the group comprising 4-(4-Fluorophenyl)-2-(4-methylsulfinylphenyl)-5-(4-pyridyl)-1H-imidazole (SB203580) and, 5-Isoquinolinesulfonamide (H-89).

The disclosure also relates to said dual-specificity tyrosine-(Y)-phosphorylation regulated kinase (Dyrkla-b 4) inhibitor selected from the group comprising 6-[2-Amino-4-oxo-4*H*-thiazol-(5Z)-ylidenemethyl]-4-(tetrahydro-pyran-4-yloxy)-quinoline-3-carbonitrile.

The disclosure also relates to said smoothened activator which is Purmorphamine (2-(1-Naphthoxy)-6-(4-morpholinoanilino)-9-cyclohexylpurine.

Examples of modulators of the interaction between β-catenin (or γ-catenin) and the coactivator proteins CBP (CREB binding protein) and p300 (E1A binding protein p300) are IQ-1 (2-(4-Acetyl-phenylazo)-2-[3,3-dimethyl-3,4-dihydro-2*H*-isoquinolin-(1E)-ylidene]-acetamide, and ICG-001((6S,9aS)-6-(4-Hydroxy-benzyl)-8-naphthalen-1-ylmethyl-4,7-dioxo-hexahydro-pyrazino[1,2-*a*]pyrimidine-1-carboxylic acidbenzylamide (WO 2007056593).

In one embodiment said priming medium is a serum free medium supplemented with insulin, transferrin and progesterone. In one embodiment said serum free medium is supplemented with 10-50 µg/ ml insulin, 10-100 µg/ ml transferrin and 10-50 nM progesterone, preferably 30-50 µg/ ml insulin, 20-50 µg/ ml transferrin and 10-30 nM progesterone. Examples of serum-free media suitable for priming are N2B27 medium (N2B27 is a 1:1 mixture of DMEM/F12 (Gibco, Paisley, UK) supplemented with N2 and B27 (both from Gibco)), N3 medium (composed of DMEM/F12 (Gibco, Paisley, UK), 25 µg/ ml insulin, 50 µg/ ml transferrin, 30 nM sodium selenite, 20 nM progesterone, 100 nM putrescine (Sigma)), or NeuroCult® NS-A Proliferation medium (Stemcell Technologies). said the disclosure also relates to a priming medium which is a serum free medium supplemented with insulin, transferrin, progesterone and a small molecule that activates the Beta-Catenin (cadherin-associated protein, beta 1; human gene name CTNNB1) pathway and/or the Wnt receptor signaling pathway and/or hedgehog (HH) signaling pathway. Preferably said small molecule is selected from the group comprising 3-(2,4-Dichlorophenyl)-4-(1-methyl-1H-indol-3-yl)-1H-pyrrole-2,5-dione (SB216763), 3-[(3-Chloro-4-hydroxyphenyl)amino]-4-(2-nitrophenyl)-1H-pyrrol-2,5-dione (SB415286), *N*⁶-{2-[4-(2,4-Dichloro-phenyl)-5-imidazol-1-yl-pyrimidin-2-ylamino]-ethyl}-3-nitro-pyridine-2,6-diamine 2HCl, 3-Imidazo[1,2-*a*]pyridin-3-yl-4-[2-(morpholine-4-carbonyl)-1,2,3,4-tetrahydro-[1,4]diazepino[6,7,1-*hi*]indol-7-yl]-pyrrole-2,5-dione, 9-Bromo-7,12-dihydro-indolo[3,2-d][1]benzazepin-6(5H)-one (Kenpaullone), 9-Bromo-7,12-dihydro-pyrido[3',2':2,3]azepino[4,5-b]indol-6(5H)-one (CHIR99021), 3-(3-Aminophenyl)-4-(1-methyl-1H-indol-3-yl)-pyrrole-2,5-dione (CP21R7, also referred to as "compound 21" herein). The disclosure also relates to a priming medium wherein said small molecule is selected from benzothiazole, 3-Fluoro-*N*-[1-isopropyl-6-(1-methyl-piperidin-4-yloxy)-1,3-dihydro-benzoimidazol-(2E)-ylidene]-5-(4-methyl-1*H*-pyrazole-3-sulfonyl)-benzamide, 4-(4-Fluorophenyl)-2-(4-methylsulfinylphenyl)-5-(4-pyridyl)-1H-imidazole (SB203580), 5-Isoquinolinesulfonamide (H-89), 6-[2-Amino-4-oxo-4*H*-thiazol-(5Z)-ylidenemethyl]-4-(tetrahydro-pyran-4-yloxy)-quinoline-3-carbonitrile, 2-(1-Naphthoxy)-6-(4-morpholinoanilino)-9-cyclohexylpurine (Purmorphamine), 2-(4-Acetyl-phenylazo)-2-[3,3-dimethyl-3,4-dihydro-2H-isoquinolin-(1E)-ylidene]-acetamide (IQ-1), and ICG-001 ((6S,9aS)-6-(4-Hydroxy-benzyl)-8-naphthalen-1-ylmethyl-4,7-dioxo-hexahydro-pyrazino[1,2-a]pyrimidine-1-carboxylic acid benzylamide.

In one preferred embodiment the priming medium is a serum- free medium containing 10-50 µg/ ml insulin, 10-100 µg/ ml transferrin and 10-50 nM progesterone supplemented with 0.5-4 µM CP21R7 (3-(3-Amino-phenyl)-4-(1-methyl-1H-indol-3-yl)-pyrrole-2,5-dione).

In one embodiment said priming medium additionally comprises recombinant bone morphogenic protein-4 (BMP4).

In one preferred embodiment the priming medium is a serum- free medium containing 10-50 µg/ ml insulin, 10-100 µg/ ml transferrin and 10-50 nM progesterone supplemented with 0.5-4 µM CP21R7 (3-(3-Amino-phenyl)-4-(1-methyl-1H-indol-3-yl)-pyrrole-2,5-dione) and 10-50 ng/ ml recombinant bone morphogenic protein-4 (BMP4).

In one embodiment step b) of the method described above comprises incubating said cells in a priming medium for at least 3 days (72 hours).

In one embodiment step b) of the method described above comprises incubating said cells in a priming medium for 2 to 4 days (48 hours to 96 hours).

In another embodiment step b) of the method described above comprises incubating said cells in a priming medium, wherein said priming medium is a serum-free medium supplemented with CP21R7 (3-(3-Amino-phenyl)-4-(1-methyl-1H-indol-3-yl)-pyrrole-2,5-dione). Preferably said priming medium is supplemented with 0.5 - 4 µM CP21R7 (3-(3-Amino-phenyl)-4-(1-methyl-1H-indol-3-yl)-pyrrole-2,5-dione), most preferably 1-2 µM CP21R7 (3-(3-Aminophenyl)-4-(1-methyl-1H-indol-3-yl)-pyrrole-2,5-dione).

In one embodiment said priming medium additionally comprises recombinant bone morphogenic protein-4 (BMP4).

In another embodiment step b) of the method described above comprises incubating said cells in a priming medium, wherein said priming medium is a serum-free medium supplemented with CP21R7 (3-(3-Amino-phenyl)-4-(1-methyl-1H-indol-3-yl)-pyrrole-2,5-dione), and incubating said cells for three days (72 hours).

In one embodiment said priming medium additionally comprises recombinant bone morphogenic protein-4 (BMP4).

In another embodiment step b) of the method described above comprises incubating said cells in a priming medium, wherein said priming medium is a serum-free medium supplemented with CP21R7 (3-(3-Amino-phenyl)-4-(1-methyl-1H-indol-3-yl)-pyrrole-2,5-dione), and growing said cells for 2 to 4 days (48 hours to 96 hours).

In one embodiment said priming medium additionally comprises recombinant bone morphogenic protein-4 (BMP4).

"Induction medium" as used herein refers to any chemically defined medium useful for the induction of primed cells into CD144 positive (CD144+) endothelial cells or PDGF-Receptor beta positive (CD140b+) vascular smooth muscle cells or a common progenitor cell type on a monolayer.

For predominant induction of endothelial cells, said induction medium is supplemented with VEGF (=Vascular endothelial growth factor) or placenta-like growth factor 1 (PLGF-1) and a small molecule adenylate cyclase activator. In one embodiment said small molecule adenylate cyclase activator leads to the activation of PKA/PKI signaling pathway. In one embodiment, said small molecule adenylate activators are chosen from the group comprising Forskolin ((3R)-(6aalphaH)Dodecahydro-6beta, 10alpha, 10balpha-trihydroxy-3beta,4abeta,7,7,10abeta-pentamethyl-1-oxo-3-vinyl-1H-naphtho[2,1-b]pyran-5beta-yl acetate), 8-Bromo-cAMP (8-Bromoadenosine-3',5'-cyclic monophosphate) and Adrenomedullin. In one embodiment said induction medium is a serum free medium supplemented with human serum albumin, ethanolamine, transferrin, insulin and hydrocortisone. Examples of serum-free media suitable for the induction are StemPro-34 (Invitrogen, principal components: human serum albumin, lipid agents such as Human Ex-Cyte® and ethanolamine or a mixture thereof, human zinc insulin, hydrocortisone, iron-saturated transferring 2-mercaptoethanol, and D,L-tocopherol acetate, or derivatives or mixtures thereof) and X-VIVO 10 and 15 (Lonza).

In one embodiment, said induction medium is a serum-free medium supplemented with human serum albumin, ethanolamine, transferrin, insulin and hydrocortisone, and 1-10 µM Forskolin and 5-100 ng/ml VEGF-A. In another embodiment, the induction medium comprises StemPro-34 (from Invitrogen) supplemented with VEGF-A 30-70 ng/ml or placenta-like growth factor 1 (PLGF-1) 30-70 ng/ml.

In one embodiment step c) of the method described above comprises inducing the differentiation into endothelial cells by incubating said primed cells in an induction medium supplemented with VEGF-A or placenta-like growth factor 1 (PLGF-1) and a small molecule adenylate cyclase activator, wherein said small molecule adenylate cyclase activator is selected from the group of Forskolin, 8-Bromo-cAMP and Adrenomedullin.

In another embodiment step c) of the method described above comprises inducing the differentiation into endothelial cells by incubating said primed cells in an induction medium supplemented with VEGF-A or placenta-like growth factor 1 (PLGF-1) and a small molecule adenylate cyclase activator for one day.

In another embodiment step c) of the method described above comprises inducing the differentiation into endothelial cells by incubating said primed cells in an induction medium supplemented with VEGF-A or placenta-like growth factor 1 (PLGF-1) and a small molecule adenylate cyclase activator for 18 hours to 48 hours, preferably for 22 hours to 36 hours.

In another embodiment step c) of the method described above comprises inducing the differentiation into endothelial cells by incubating said primed cells for one day in an induction medium supplemented with VEGF-A or placenta-like growth factor 1 (PLGF-1) and a small molecule adenylate cyclase activator for one day, wherein said small molecule adenylate cyclase activator is selected from the group of Forskolin, 8-Bromo-cAMP and Adrenomedullin.

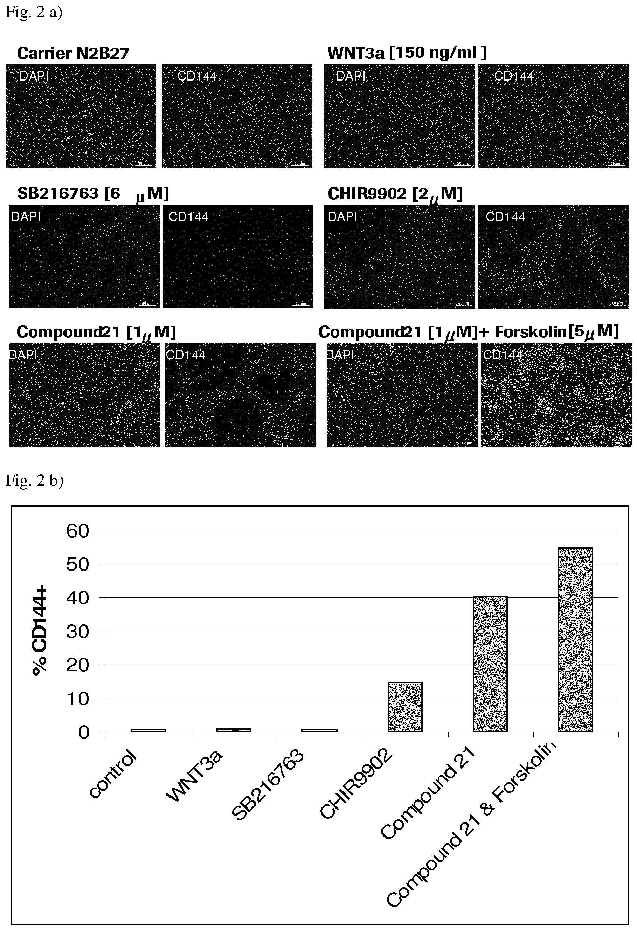

With the method presented in this invention it is now possible to differentiate endothelial cells from pluripotent stem cells with a yield of up to 85 % (Figure 2). The product of step c) can be easily identified in a cell culture as CD 144+ cells.

For a predominant induction of vascular smooth muscle cells, step c) of the method described above comprises the incubation of said primed cells in a VSMC induction medium for 18-48h, preferably for 22-36 hours. VSMC induction medium is a chemically defined medium supplemented with growth factors and/or small molecules enhancing the formation and survival of vascular smooth muscle cells.

In one embodiment step the induction medium is a chemically defined serum replacement medium (SR medium). SR medium comprises of a basic medium (e.g. RPMI medium, Invitrogen, cat num. 11835-063, or DMEM medium) supplemented with a chemically defined serum replacement (2-15%), e.g. Knock-out serum replacement from Invitrogen, Catalogue number10828028. It may further comprise stable glutamine, non-essential amino acids and betamercaptoethanol.

In another embodiment, the induction medium medium is supplemented with IWR1 (Inhibitor of Wnt response 1) in a range from 1-5µM.

In another embodiment, the induction medium comprises of a serum-free medium supplemented with 2-20 ng/ml of an activator of the platelet derived growth factor (PDGF) signaling pathway and 2-10 ng/ml of an activator of the TGF beta signaling pathway. Examples of activators of PDGF signaling comprise e.g. PDGF-AA, PDGF-BB (e.g. RnD, Cat. Num. 220-BB), and PDGF-AB. One exemplary activators of TGFbeta signaling is ActivinA (e.g. RnD, Cat. Num. 338-AC). Examples of serum-free media suitable for the induction are N2B27 medium (N2B27 is a 1:1 mixture of DMEM/F12 (Gibco, Paisley, UK) supplemented with N2 and B27 (both from Gibco), StemPro-34 (Invitrogen, principal components: human serum albumin, lipid agents such as Human Ex-Cyte® and ethanolamine or a mixture thereof, human zinc insulin, hydrocortisone, iron-saturated transferring 2-mercaptoethanol, and D,L-tocopherol acetate, or derivatives or mixtures thereof) and X-VIVO 10 and 15 (Lonza). In a preferred embodiment said serum-free medium useful for prevalent induction of vascular smooth muscle cells is N2B27 medium.

In another embodiment, the induction medium comprises of a serum-free medium supplemented with 2-20 ng/ml of an activator of the platelet derived growth factor (PDGF) signaling pathway and 2-10 ng/ml of an activator of the TGF beta signaling pathway, and further comprises human serum albumin, ethanolamine, transferrin, insulin and hydrocortisone.

In another embodiment step c) of the method described above comprises inducing the differentiation into smooth vascular muscle cells by incubating said primed cells in an induction medium as described above for one day.

In another embodiment step c) of the method described above comprises inducing the differentiation into smooth vascular muscle cells by incubating said primed cells in an induction medium as described above for 18 hours to 48 hours, preferably for 22 hours to 36 hours.

With the method presented in this invention it is now possible to differentiate smooth vascular muscle cells from pluripotent stem cells with a yield of 90 %. The product of step c) can be easily identified in a cell culture as CD140b+ cells.

In a further embodiment, said method additionally comprises step
d) incubating the product of step c) under conditions suitable for proliferation of the endothelial cells or vascular smooth muscle cells.

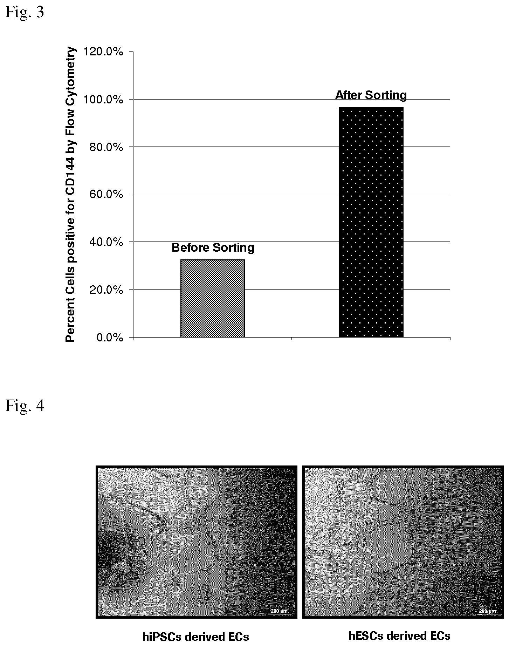

Preferably said conditions suitable for proliferation of the endothelial cells comprise harvesting of said CD 144+ cells and expanding them in a chemically defined expansion medium. "Harvesting" as used herein relates to the enzymatical dissociation of the cells from the adhesive substrate and subsequent resuspension in new medium. In one preferred embodiment, cells are sorted after harvesting. Cell sorting can be achieved through methods known in the art, e.g. by magnetic-activated cell sorting (MACS) (Figure 3) or a flow cytometry-activated cell sorting (FACS) separation. "Expansion medium" as used herein refers to any chemically defined medium useful for the expansion and passaging of CD 144+ endothelial cells on a monolayer. In one embodiment said expansion medium is a serum free medium supplemented with VEGF-A. Examples of serum-free media suitable for the expansion of endothelial cells are StemPro-34 (Invitrogen), EGM2 (Lonza) and DMEM/F12 (Invitrogen) supplemented with 8ng/ml FGF-2, 50 ng/ml VEGF and 10 µM SB431542 (4-(4-Benzo[1,3]dioxol-5-yl-5-pyridin-2-yl-1*H*-imidazol-2-yl)-benzamide). Preferably, the endothelial cells are cultured in adherent culturing conditions. In one embodiment, the expansion medium is supplemented with 5-100 ng/ml VEGF-A. In another embodiment, the expansion medium is StemPro-34 supplemented with 5-100 ng/ml VEGF-A.

The endothelial cells obtained by the method described herein can be expanded for several passages and culturing is well characterized. It is possible to freeze and thaw the aliquots of the endothelial cells obtained by the method described herein reproducibly.

Preferably said conditions for proliferation of VSMCs comprise harvesting of said CD140b+ cells and expanding them in a chemically defined expansion medium. "Harvesting" as used herein relates to the enzymatical dissociation of the cells from the adhesive substrate and subsequent resuspension in new medium. In one preferred embodiment, cells are sorted after harvesting either by positive selection for CD140b+ VSMCs or depletion of CD 144+ endothelial cells. Cell sorting can be achieved through methods known in the art, e.g. by magnetic-activated cell sorting (MACS) (Figure 3) or a flow cytometry-activated cell sorting (FACS) separation. "Expansion medium" as used herein refers to any chemically defined medium useful for the expansion and passaging of CD140b+ VSMCs on a monolayer. In one embodiment said expansion medium is identical to described VSMC induction medium. In another embodiment expansion medium is a serum-free medium supplemented with EGF (5-20ng/ml) and FGF2 (5-20ng/ml). Examples of serum-free media suitable for the expansion of VSMCs are StemPro-34 (Invitrogen), DMEM/F12 (Invitrogen) and DMEM (Invitrogen), or previously described N2B27. In another embodiment expansion medium is said SR medium. Preferably, VSMCs are cultured in adherent culturing conditions.

VSMCs obtained by the method described herein can be expanded for several passages and culturing is well characterized.

In one embodiment of the present invention a method for generating patient specific or healthy individual specific vascular bed cells is provided. Towards this end, human induced pluripotent stem cells (iPSCs) obtained from a patient or healthy individual are used in the method described herein. Said patient-specific human iPSCs can be obtained by methods known in the art by reprogramming somatic cells obtained from the patients or healthy individuals to pluripotent stem cells. For example, fibroblast cells, keratinocytes or adipocytes may be obtained by skin biopsy from the individual in need of treatment or from a healthy individual and reprogrammed to induced pluripotent stem cells by the methods known in the art. Other somatic cells suitable as a source for induced pluripotent stem cells are leucocytes cells obtained from blood samples or epithelial cells or other cells obtained from urine samples. The patient specific induced pluripotent stem cells are then differentiated to patient specific or healthy individual specific endothelial cells or vascular smooth muscle cells by the method described herein. The disclosure also relates to a population of endothelial cells or vascular smooth muscle cells produced by any of the foregoing methods. Preferably, the population of endothelial cells or vascular smooth muscle cells is patient specific, i.e. derived from iPSCs obtained from diseased individuals. Also disclosed are said population of endothelial cells or vascular smooth muscle cells which are obtained from a healthy individual.

Patient derived endothelial cells and vascular smooth muscle cells represent a disease relevant *in vitro* model to study the pathophysiology of vascular complications for diseases like Diabetes Type-2 and Type-1, Metabolic Syndrome and Severe Obesity. The disclosure also relates to the endothelial cells and / or vascular smooth muscle cells obtained by this method used for screening for compounds that reverse, inhibit or prevent vascular complications caused by dysfunction of endothelial cells, e.g. of vascular complications caused by diabetes Type-2 and Type-1, Metabolic Syndrome, Severe Obesity, Hypercholesterolemia, Hypertension, coronary artery disease, nephropathy, retinopathy, kidney failure, tissue ischemia, chronic hypoxia, artherosclerosis and tissue edema caused by drug-induced toxicity. Preferably, said endothelial cells and/ or vascular smooth muscle cells obtained by the method of the invention described herein are derived from diseased subjects. In another embodiment the endothelial cells and / or vascular smooth muscle cells obtained by this method are used for screening and evaluating drug-induced tissue edema. Preferably, said endothelial cells and / or vascular smooth muscle cells obtained by the method of the invention described herein are derived from individuals affected by idiosyncratic drug-induced tissue edema. Differentiating endothelial cells and / or vascular smooth muscle cells from diseased subjects represents a unique opportunity to early evaluate drug safety in a human background paradigm. In another embodiment the endothelial cells obtained by this method are used as an *in vitro* model of the blood brain barrier.

The present invention provides a highly efficient method to supply patient specific vascular bed cells or compatible cells from healthy individuals with the same HLA type suitable for transplantation, both derived in xeno-free conditions. "Xeno-free culture conditions" refers to a medium and a substrate for attachment that contains components only of human and recombinant origin. Thus the risk of contamination with xenopathogens is circumvented and the endothelial cells are safe for use in regenerative medicine. Differentiation of patient specific induced pluripotent stem cells (iPSCs) into patient specific endothelial cells and / or vascular smooth muscle cells with the method described herein represents an easy accessible and reproducible technology to generate autologous sources of endothelial cells and vascular smooth muscle cells. The use of autologous and/or compatible cells in cell therapy offers a major advantage over the use of non-autologous cells, which are likely to be subject to immunological rejection. In contrast, autologous cells are unlikely to elicit significant immunological responses.

In a further preferred aspect of the invention the generation of a BioBank of patient specific endothelial cells and / or vascular smooth muscle cells is envisaged. In one embodiment, a BioBank comprising different populations of endothelial cells and / or vascular smooth muscle cells obtained from healthy individuals and / or patients is generated. The term "BioBank" as used herein means a library of biological samples taken from different individuals or species. The archived collection of specimen and associated data is intended for research purposes with the aim of addressing diseases associated with vascular complications. In another embodiment, said BioBank is used for vascular regenerative medicine approaches.

The disclosure also relates to a therapeutic composition comprising endothelial cells and / or vascular smooth muscle cells produced by any of the foregoing methods or comprising any of the foregoing cell populations. Preferably, the therapeutic compositions further comprise a physiologically compatible solution including, for example, a phosphate-buffered saline with 5% human serum albumin. Said therapeutic composition can be used to treat, prevent, or stabilize diseases associated with vascular complications such as for example, vascular complications caused by diabetes Type-2 and Type-1, Metabolic Syndrome, Severe Obesity, Hypercholesterolemia, Hypertension, coronary artery disease, nephropathy, retinopathy, kidney failure, tissue ischemia, chronic hypoxia, artherosclerosis and tissue edema caused by drug-induced toxicity, to recover functions after a stroke or as a carrier to secrete factors into blood. For example, fibroblast cells, keratinocytes or adipocytes may be obtained by skin biopsy from the individual in need of treatment or from a healthy individual and reprogrammed to induced pluripotent stem cells by the methods known in the art ("Induction of pluripotent stem cells from adult human fibroblasts by defined factors." Takahashi et al., 2007, Cell 131, 861-72). Other somatic cells suitable as a source for induced pluripotent stem cells are leucocytes cells obtained from blood samples or epithelial cells or other cells obtained from urine samples. The patient specific induced pluripotent stem cells are then differentiated to endothelial cells by the method described herein, harvested and introduced into the individual to treat the condition. The endothelial cells and / or vascular smooth muscle cells produced by the method of the invention may be used to replace or assist the normal function of diseased or damaged tissue.

The disclosure also relates to use of BioBanks of endothelial cells and / or vascular smooth muscle cells for therapy of diseases associated with vascular complications. The BioBanks preferably comprise endothelial cells and / or vascular smooth muscle cells obtained from patients or healthy individuals with several HLA types. Transplanting cells obtained from a healthy donor to an individual in need of treatment with a compatible HLA type obviates the significant problem of rejection reactions normally associated with heterologous cell transplants. Conventionally, rejection is prevented or reduced by the administration of immunosuppressants or anti-rejection drugs such as cyclosporine. However, such drugs have significant adverse side-effects, e.g., immunosuppression, carcinogenic properties, kidney toxicity as well as being very expensive. The use disclosed herein eliminates, or at least significantly reduces, the need for anti-rejection drugs, such as cyclosporine, imulan, FK-506, glucocorticoids, and rapamycin, and derivatives thereof.

With respect to the therapeutic methods disclosed herein, it is not intended that the administration of endothelial cells and / or vascular smooth muscle cells to a mammal be limited to a particular mode of administration, dosage, or frequency of dosing; the disclosure contemplates all modes of administration, including intramuscular, intravenous, intrarticular, intralesional, subcutaneous, or any other route sufficient to provide a dose adequate to prevent or treat a disease. The endothelial cells and / or vascular smooth muscle cells may be administered to the mammal in a single dose or multiple doses. When multiple doses are administered, the doses may be separated from one another by, for example, one week, one month, one year, or ten years. One or more growth factors, hormones, interleukins, cytokines, small molecules or other cells may also be administered before, during, or after administration of the cells to further bias them towards a particular cell type.

As used herein the term "differentiating", "differentiation" refers to one or more steps to convert a less-differentiated cell into a somatic cell, for example to convert a pluripotent stem cell into a vascular bed cell. Differentiation of a pluripotent stem cell to a vascular bed cell, i.e. into endothelial cells and / or vascular smooth muscle cells is achieved by the method described herein.

The term "stem cell" as used herein refers to a cell that has the ability for self-renewal. An "undifferentiated stem cell" as used herein refers to a stem cell that has the ability to differentiate into a diverse range of cell types. As used herein, "pluripotent stem cells" as used herein refers to a stem cell that can give rise to cells of multiple cell types. Pluripotent stem cells (PSCs) include human embryonic stem cells (hESCs) and human induced pluripotent stem cells (hiPSCs). Human induced pluripotent stem cells can be derived from reprogrammed somatic cells, e.g. by transduction of four defined factors (Sox2, Oct4, Klf4, c-Myc) by methods known in the art. Said human somatic cells can be obtained from a healthy individual or from a patient. These donor cells can be easily obtained from any suitable source. Preferred herein are sources that allow isolation of donor cells without invasive procedures on the human body, for example human skin cells, blood cells or cells obtainable from urine samples. Although human pluripotent stem cells are preferred, the method is also applicable to non-human pluripotent stem cells, such as primate, rodent (e.g. rat, mouse, rabbit) and dog pluripotent stem cells.

As used herein, "vascular bed cells" are cells that form a vascular vessel in vivo. Vascular vessels consist of an inner lacer named intima layer formed by endothelial cells and an adjacent layer termed media formed by smooth muscle cells Examples of vascular bed cells are endothelial cells (CD 144+) and vascular smooth muscle cells (CD140b+).

As used herein, "endothelial cells" are cells that express the specific surface marker CD 144 (Cluster of Differentiation 144, also known as Cadherin 5, type 2 or vascular endothelial (VE)-cadherin, official symbol CDH5) and possess characteristics of endothelial cells, namely capillary-like tube formation, and the expression of one or more further surface markers selected from the group of, CD31 (Cluster of Differentiation 31, official symbol PECAM1), vWF (Von Willebrand factor, official symbol VWF), CD34 (Cluster of Differentiation 34, official symbol CD34), CD105 (Cluster of Differentiation 105, official symbol ENG), CD146 (Cluster of Differentiation 34, official symbol MCAM), and VEGFR-2 (kinase insert domain receptor (a type III receptor tyrosine kinase), official symbol KDR).

As used herein, "Vascular smooth muscle cells" are cells that express the specific surface marker CD140b (Cluster of Differentiation 140b, also known as PDGF-Receptor beta) and possess characteristics of vascular smooth muscle cells, namely contractile and/or secretory functions and the expression of one or more further proteins selected from the group of SMA (alpha-smooth muscle actin), SM22alpha, smooth muscle myosin heavy chain, CRBP1, and Smemb.As used herein, "diseases associated with vascular complications" relates to any disease caused by injury, activation or dysfunction of endothelial cells and/or vascular smooth muscle cells. Examples for diseases associated with vascular complications are diabetes Type-2 and Type-1, Metabolic Syndrome, Severe Obesity, Hypercholesterolemia, Hypertension, coronary artery disease, nephropathy, retinopathy, kidney failure, tissue ischemia, chronic hypoxia, artherosclerosis and tissue edema caused by drug-induced toxicity.

### Short description of the figures

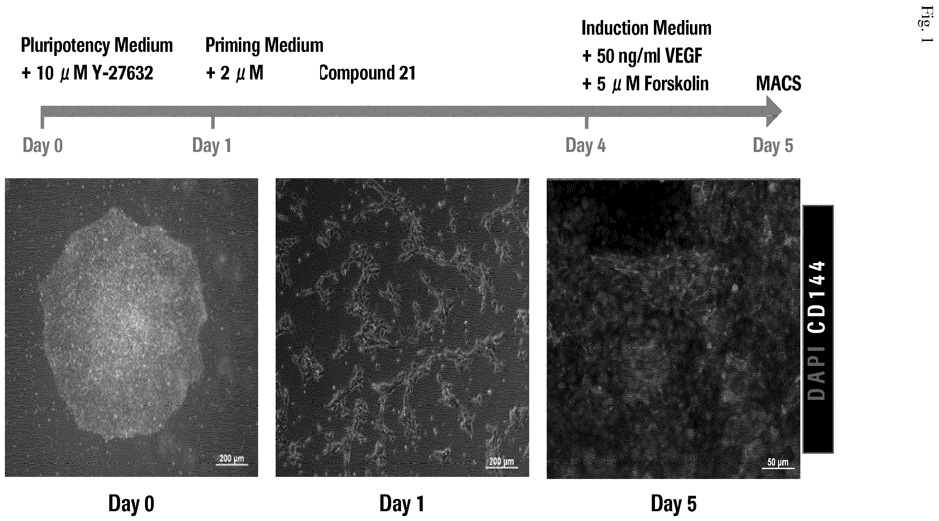

Figure 1: Schematic representation of the method for differentiating human pluripotent stem cells (PSCs) to endothelial cells CD144+. Day 0: human PSCs were enzymatically dissociated and plated on pre-coated matrigel plates using a concentration of 35000 cells/cm² in pluripotency medium (mTeSR2 with Y27631 10 µM). Day 1: Media change with fresh priming medium (N2B27 with Compound 21 (CP21R7) 2 µM). Day 4: Media change with fresh induction medium (StemPro-34 with Forskolin 5µM and VEGF-A 50 ng/ml). At Day 5 CD144+ endothelial cells are shown and sorted by MACS for further expansion.
Figure 2: a) Quantification CD144+ Stem Cell-Derived Endothelial Cells by image based high content analysis (HCA). Human Embryonic Stem cells have been differentiated in monolayer conditions with different small molecules. Upper and middle panel: Day 1 priming medium with different compounds; Day 4 induction medium + VEGF-A 50 ng/ml. Carrier N2B27 alone (upper panel, left); Wnt3a 150 ng/ml added to the priming medium (upper panel, right); SB216763 6 µM added to the priming medium (middle panel, left), CHIR 9902 2 µM added to the priming medium (middle panel, right). Lower panels: Day 1 priming medium with 2 µM Compound 21 (CP21R7); Day 4 induction medium + VEGF-A 50 ng/ml (lower panel, left) and Day 4 induction medium + VEGF-A 50 ng/ml + Forskolin 5 µM (lower panel, right). DAPI (cell nuclei) CD144 (membrane staining). **b) Quantification graph:** Percent CD144+ positive cells.
**Figure 3****:** Flow Cytometry analysis of the pluripotent stem cell-derived CD144+ endothelial cells. Endothelial cells differentiated in monolayer conditions (priming medium 2 µM CP21R7, induction medium VEGF-A 50ng/ml and Forskolin 5 µM) were analyzed before and after MACS sorting for the CD144+ expression. Before MACS Sorting 32.5% CD144+ endothelial cells and after MACS sorting 96.5% CD144+ endothelial cells.
**Figure 4****:** Tube formation in angiogenesis assay. hESCs and hiPSCs have been differentiated in monolayer conditions (priming medium 2 µM CP21R7, induction medium VEGF-A 50 ng/ml and Forskolin 5µM) and after MACS sorting tested for the tube formation in the angiogenesis assay. Coverage of the well with tube structures: hESCs-derived endothelial cells 90%, hiPSCs-derived endothelial cells 80% (**quantification graph**). Representative pictures of the tube structures: hESCs-derived endothelial cells (**left photo**), hiPSCs-derived endothelial cells (**right photo**).

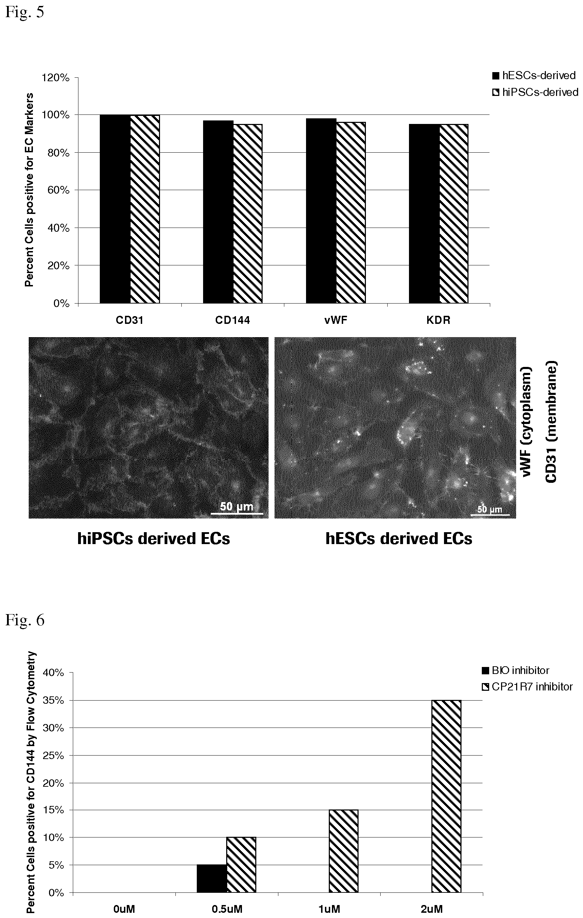

**Figure 5****:** Characterization of monolayer differentiated hPSCs-derived endothelial cells after sorting. hESCs and hiPSCs have been differentiated in monolayer conditions (priming medium 2 µM CP21R7, induction medium VEGF-A 50ng/ml and Forskolin 5 µM) and after MACS sorting tested for the expression of the endothelial cells markers CD31, vWF, CD 144, VEGFR-2 (KDR) by immunocytochemistry analysis and flow cytometry analysis. **Quantification graph:** hESCs-derived endothelial cells (black bars), hiPSCs-derived endothelial cells (black and white bars). Representative pictures CD31/vWF/DAPI immunocytochemistry: hiPSCs-derived endothelial cells (**left photo**), hESCs-derived endothelial cells (**right photo**).
**Figure 6****:** Direct comparison BIO versus CP21R7 to differentiate hESCs into endothelial cells CD144+. hESCs have been differentiated in monolayer conditions in parallel using different concentrations at Day 1 (0 µM, 0.5 µM, 1 µM, 2 µM) for the BIO or for the COMPOUND 21 (CP21R7) as supplement to the carrier medium N2B27, followed at Day 4 with induction medium VEGF-A 50ng/ml and Forskolin 5 µM for all the conditions. Using BIO at 0.5 µM we could reach the maximum expression of the CD144 (5%) and the other concentrations were toxic to the cells. The CP21R7 showed a clear dose response regarding CD144 expression (up to 35%) and any toxicity at the tested concentrations.

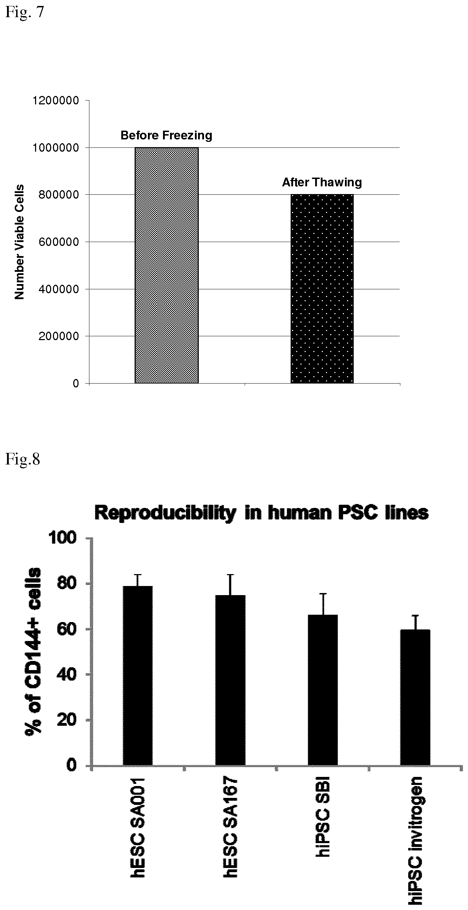

**Figure 7****:** Viability of cryopreserved PSCs-derived endothelial cellss. 1x10⁶ hESCs-derived endothelial cells have been cryopreserved at day 5 after MACS sorting for the CD 144 expression. 8x10⁵ hESCs-derived endothelial cells (80% of the total frozen cell number) were viable after thawing.
**Figure 8****:** Reproducibility of the endothelial cell differentiation method. Flow cytometric Quantification of CD144+ stem cell-derived endothelial cells at day 6. Two human embryonic stem cell lines (SA001 and SA167 from Cellartis) and two induced pluripotent stem cell lines (SBI System bioscience and Life technologies) were differentiated resulting in average efficiency between 60 to 80% CD144+ endothelial cells.

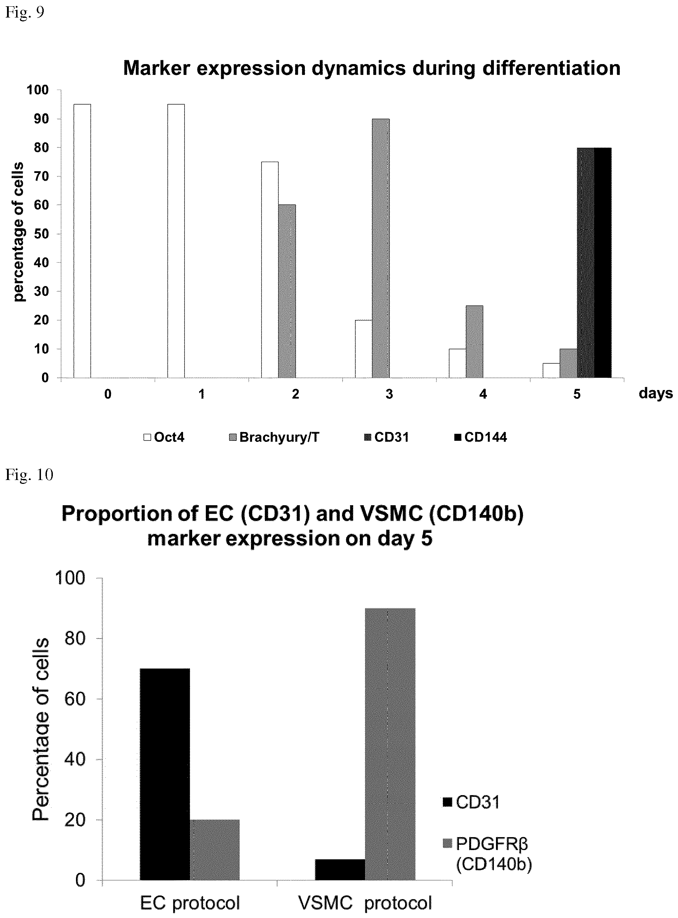

**Figure 9****:** Expression analysis of the markers Oct4 (pluripotency), Brachyury/T (pan-mesoderm), CD31 and CD 144 (endothelial cells) over the course of time in the differentiation. Pluripotent stem cells differentiate in priming medium (day 2-4) into mesodermal progenitor cells by losing Oct4 (white bars) and gaining Brachyury/T (grey bars) expression. Upon media change with EC induction medium (upon day 4) mesodermal progenitor cells differentiate further into endothelial cells upregulating CD31 (dark grey bar) and CD 144 (black bar) expression. These findings were confirmed by whole genome expression profiling (data not shown).
**Figure 10****:** Flow Cytometric Analysis of endothelial cell-specific (CD31) and vascular smooth muscle cell-specific (CD140b) marker expression at day 5 of the differentiation methods. Depicted here is the proportion of ECs (black bars) and VSMCs (grey bars) of cells differentiated first with priming medium and then either with EC induction medium (StemPro-34 with VEGF 200 ng/ml and Forskolin 2 µM) or VCMC induction medium (RPMI with 10% Knockout Serum Replacement). Proportion of ECs and VSMC can be modulated by fine-tuning the differentiation system, which allows to shift lineage commitment either predominantly into endothelial (∼70%) or vascular smooth muscle cells (∼90%).

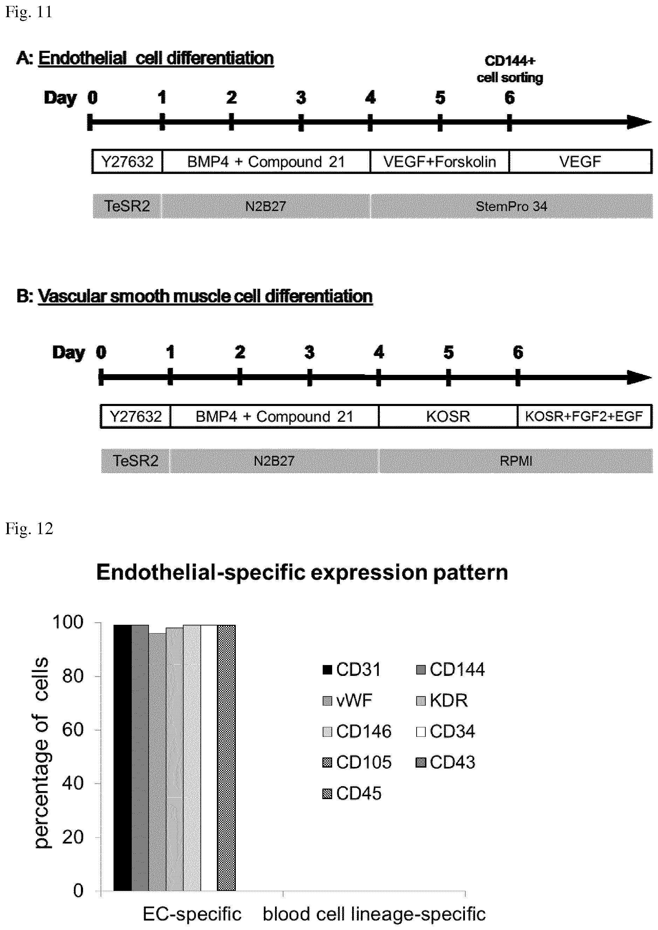

**Figure 11****:** Schematic representation of the method for differentiating human pluripotent stem cells (PSCs) to endothelial cells (A) or to vascular smooth muscle cells (B). From day 0 until day 4 the protocol for both target cells is identical. Day 0: human PSCs were enzymatically dissociated and plated on pre-coated matrigel plates using a concentration of 35000 cells/cm² in pluripotency medium (TeSR2 with Y27631 10 µM). Day 1: Media change with fresh priming medium (N2B27 with Compound 21 (CP21R7) 1 µM and recombinant bone morphogenic protein-4 (BMP4) 25ng/ml). A) Day 4: Media change with fresh EC induction medium (StemPro-34 with Forskolin 2 µM and 200 ng/ml VEGF) for endothelial cells. At day 6 endothelial cells (CD 144+) are purified by MACs preparation. Sorted endothelial cells are plated on pre-coated fibronectin plates and maintained in EC expansion medium (StemPro-34 with 50 ng/ml VEGF). B) Day 4, Media change with fresh VSMC induction medium (RPMI with 10% Knockout Serum Replacement) for vascular smooth muscle cells (VSMCs). At day 6 cells are enzymatically dissociated and plated on pre-coated fibronectin plates in VSMC expansion medium (RPMI with EGF 10ng/ml and FGF2 10ng/ml).
**Figure 12****:** Characterization of SC-derived ECs. MACS-purified CD144+cells were grown until confluence and then analyzed by flow cytometry. The cells possess an overall endothelial-specific expression pattern; positive for CD31, CD34, CD105, CD144, CD146, KDR, vWF (von-Willebrand factor) and negative for the hematopoietic lineage markers CD43 and CD45.

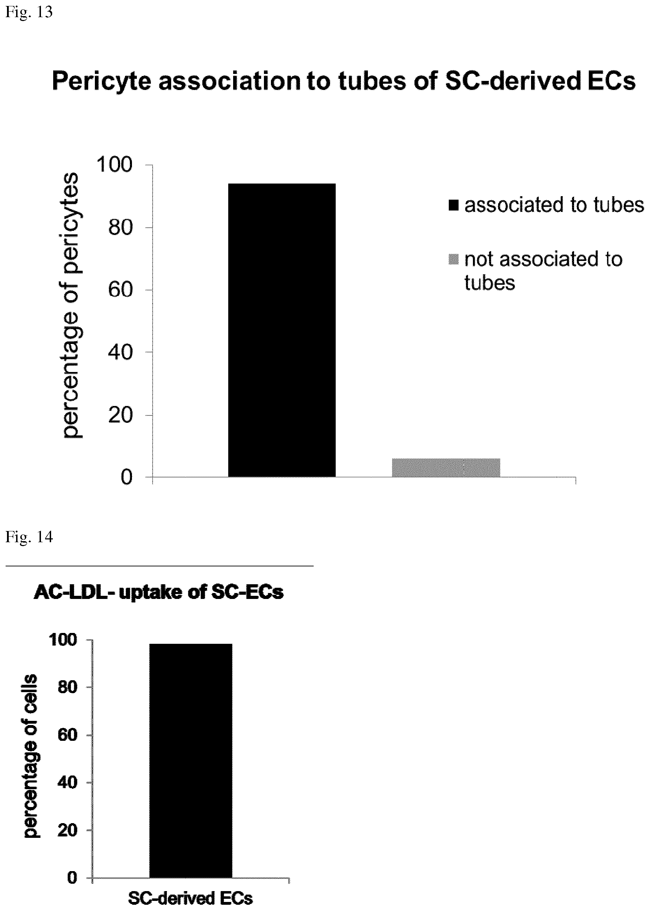

**Figure 13****:** Cellular interaction assay of vascular bed cells. Primary human brain pericytes (vascular smooth muscle cells) and Stem Cell-derived endothelial cells are co-cultured in tube formation assays. Pericytes associated to the tube-like structures, which were formed by SC-derived ECs. 94% of the seeded number of pericytes aligned or wrapped around the SC-derived endothelial cells (quantification graph).
**Figure 14****:** Cellular uptake of acetylated low-density lipoprotein (Ac-LDL) assay. Internalization was monitored by the use of fluorophor-conjugated-C (AlexaFluor594- Alexa Fluor 594). After the incubation 98% of SC-derived ECs internalized fluorophor-conjugated Ac-LDL from the growth medium (quantification graph).

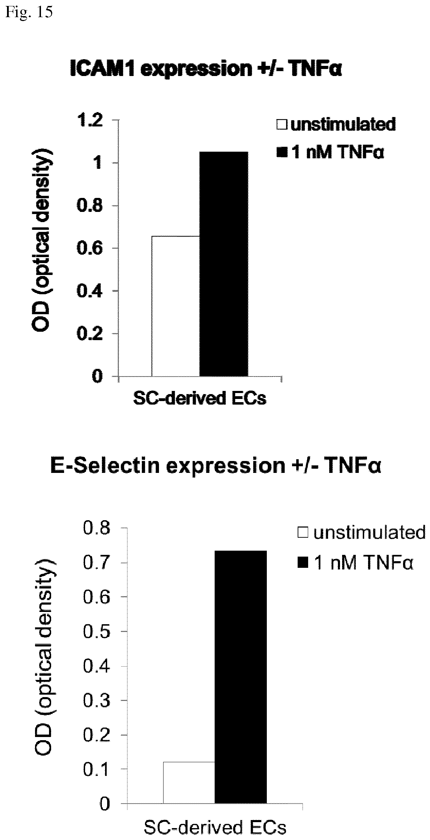

**Figure 15****:** Pro-inflammatory cytokine response assay. SC-derived ECs upregulate the expression of adhesion molecules such as ICAM1 and E-SELECTIN upon pro-inflammatory cytokine administration. Cell-based ELISA was used to measure the activation of SC-derived endothelial cells in response to 1 nM TNFα. ICAM1 and E-SELECTIN were both significantly upregulated (quantification graphs).

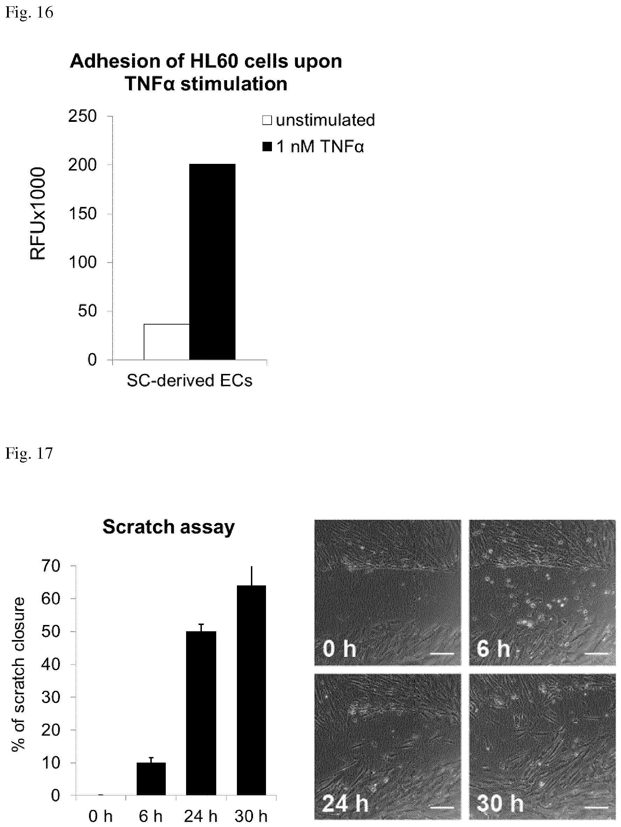

**Figure 16****:** Leukocyte-endothelial adhesion assay. Activated endothelial cells present adhesion molecules for the recruitment leukocytes. The response to 1 nM TNFα was studied by co-cultivating Calcein-stained HL60 cells with SC-derived ECs. Adhesion of HL60 cells to endothelial cells was quantified by measuring the fluorescence intensity. Recruitment of HL60 leukocytes was significantly enhanced when SC-derived ECs were stimulated with TNF-α (quantification graph).
**Figure 17****:** Scratch assay. Mural cell properties of SC-derived VSMC were assured in migration/wound healing. Over time SC-derived VCMCs extended into the wound edge as free single cells (photo panel). The scratch wide was measured at four different points (0, 6, 24 and 30 hours) average values were used to determine scratch closure (quantification graph).

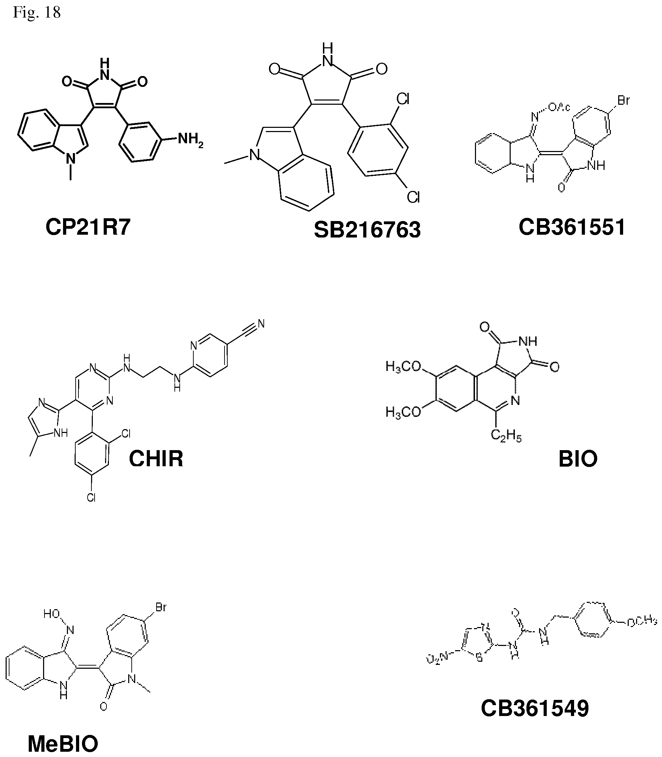

**Figure 18****:** Chemical structures of GSK3β inhibitors.

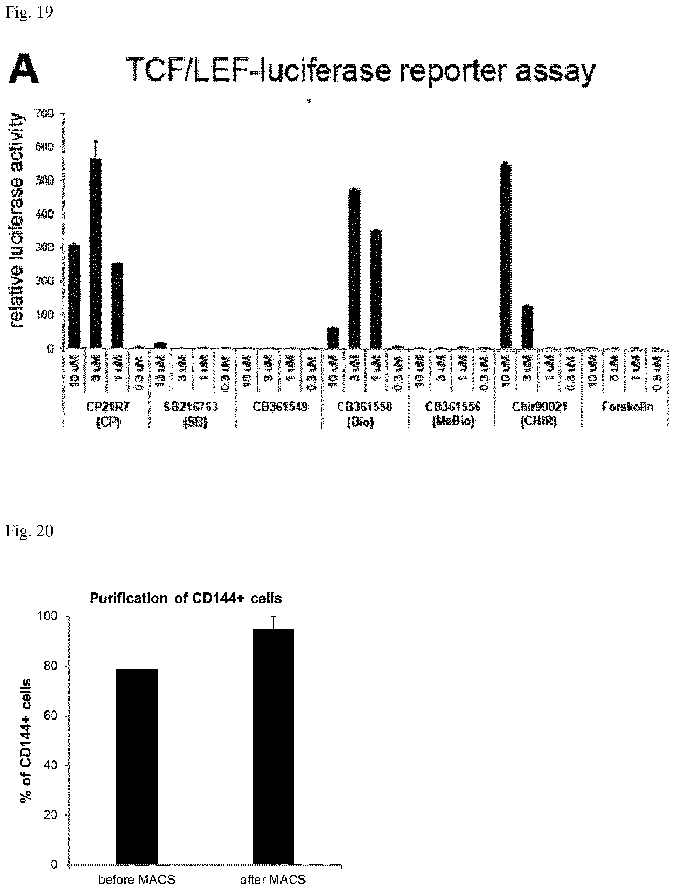

**Figure 19****:** Compound selection. TCF/LEF reporter assay in human PA-1 reporter cells (DeAlmeida et al., 2007). After the treatment of reporter cells with GSk3 inhibitors at indicated concentrations β-catenin-mediated TCF/LEF transcriptional activity was measured as luciferase activity. The Compound Cp21R7 induced highest luciferase activity at a concentration of 3 µM.

### Materials and Methods

### Cell Culture:

Pluripotency Medium: TeSR2 supplemented with Y27632 ROCK Kinase inhibitor (commercially available, e.g. Catalogue Number: 1254 from Tocris bioscience).
Priming Medium: N2B27 *(N2B27 is a 1:1 mixture of DMEM*/*F12 (Gibco, Paisley, UK) supplemented with N2 and B27 (both from Gibco)* supplemented with the Compound 21 (CP21R7), a pyrrolidindione-based GSK3 inhibitor.
CP21R7: 3-(3-Amino-phenyl)-4-(1-methyl-1H-indol-3-yl)-pyrrole-2,5-dione (also referred to as "compound 21" herein; see e.g. L. Gong et al; Bioorganic& Medicinal Chemistry Letters 20 (2010), 1693-1696).
Endothelial cells Induction Medium: StemPro-34 SFM (Invitrogen) supplemented with the Forskolin and VEGF-A.
Endothelial cells Expansion Medium: StemPro-34 (Invitrogen) supplemented with VEGF-A.
VSMC induction medium: RPMI Medium 1640 (Invitrogen) supplemented with 10% Knockout Serum Replacement (Invitrogen)
VSMC expansion medium: RPMI Medium 1640 (Invitrogen) supplemented with 10% Knockout Serum Replacement (Invitrogen) and EGF and FGF-2.

### Reference human

ESCs: SA001, LOT CA001 were isolated on March 20, 2001 at Göteborg University and Cellartis AB Arvid Wallgrens Backe 20, SE-413 46 Göteborg, SWEDEN follows all applicable laws in Sweden and is approved by the Local Research Ethics Committees at Göteborg University and Uppsala University. Embryo source: Frozen, surplus from IVF. Donor confidentiality: In order to protect the privacy and the confidentiality of the donors, all identifiers associated with the embryo donors have been removed. Thus, no information about the donors is accessible. Notably, the donation did not result in any financial gain for the donors.

Human iPSCs: Catalogue Number: SC101A-1 Lot. Number 110218-FF from SBI System Biosciences / Catalogue Number: A13777 from Life technologies Gibco® Episomal hiPSC Line.

Derivation of human vascular bed cells from hESCs. We have the approval to work with hESCs and to derive different cell lines (such as: vascular bed cells, cardiomyocytes, hepatocytes, and adipocytes). The responsible ethical committee (Ethikkommission beider Basel) and the Federal office of public health have approved our research project. (Ref-No: R-FP-S-1-0002-0000).

### Protocols:

Human pluripotent stem cells are routinely cultured on hESC-qualified Matrigel (BD Bioscience) in TeSR2 medium (Stem cell Technologies). Cultures are passaged every 4-6 days using StemPro Accutase (Invitrogen). For an increased viability TeSR2 medium is supplemented with 10 µM ROCK-inhibitor one hour prior enzymatic dissociation.

### 1. Method for differentiation of pluripotent stem cells into vascular bed cells

**(i) attachment and priming of PSCs:** Before the enzymatic dissociation of hPSC colonies using StemPro Accutase (Invitrogen) cells are preincubated for one hour with 10 µM ROCK-Inhibitor Y27632. 35.000 single hPSCs per cm² are plated onto growth factor reduced Matrigel (BD bioscience) coated cell culture plates in TeSR2 medium supplemented with 10 µM ROCK-Inhibitor. On day1 attachment medium is exchanged to N2B27 (Gibco) medium supplemented with (a) 2 µM Compound 21 (CP21R7) or (b) 1 µM Compound 21 (CP21R7) and 25 ng/ml BMP4 (R&D Systems). Cells are cultivated for additional 3 days without media change.
**(ii). Induction ECs:** On day 4 priming medium is replaced with StemPro-34 SFM medium (Invitrogen) supplemented with (a) 50 ng/ml VEGF and 5 µM Forskolin or (b) 200 ng/ml VEGF and 2 µM Forskolin. On day 5 induction medium is withdrawn and cells are used for MACS separation of CD 144+ cells.

### 2. MACS purification

During the whole protocol, cells were kept cold and pre-cooled solutions were used. After discarding the medium, the cells were washed with 5 ml PBS (Ca²⁺ and Mg²⁺ free). Next, the cells were incubated for 2-4 min at 37°C in 3 ml pre-warmed StemPro Accutase (Invitrogen). Cells were resuspended in 3 ml StemPro-34 Medium (Invitrogen) by gently pipetting up down. Cell number and viability was determined by counterstaining with trypan blue.

Cells were then centrifuged at 1000 rpm for 4 min. Cells were resuspended in MACS-buffer (0.5% BSA + 2mM EDTA in PBS), and 20 µl α-CD144-PE antibody (BD bioscience) was added per 1x10⁶ cells, and incubated for 15 min at 4°C. After addition of 8 ml StemPro-34 medium, cells were centrifuged at 1000 rpm for 4 min. The cell pellet was resuspended in MACS-buffer (0.5% BSA + 2mM EDTA in PBS) and 100 µl anti-PE Microbeads (Miltenyie Biotech) per 1x10⁷ cells were added, and incubated for 15 min at 4°C. Next, 8 ml StemPro-34 medium was added, and the cells centrifuged at 1000 rpm for 4 min. Cells were resuspended in StemPro-34 medium LS (up to 1x10⁷ per ml) and the cell suspension filtered. Next the suspension was filtered using Pre-Separationfilters 30 µm (Miltenyi Biotech). LS columns (Miltenyi Biotech) were preprepared by rinsing with 3 ml StemPro-34 medium. The filtered cell suspension was applied to the column (about 1x10⁷ cells per column), the column washed three times with 3 ml StemPro-34 medium. The cells were eluted using 5 ml StemPro-34 medium + 50ng/ml VEGF (Preprotech). Cells were seeded onto human fibronectin (BD Bioscience) coated tissue culture dishes.

Figure 2 shows the quantification of CD 144 positive stem-cell derived endothelial cells, adding different small molecules to the priming medium at day 1; and changing on day 4 to induction medium + VEGF-A 50 ng/ml. With priming medium N2B27 alone, 0.5 % of nuclear region area was CD144 positive; Wnt3a 150 ng/ml added to the priming medium resulted in 0.8 % CD144+ cells; SB216763 6 µM added to the priming medium resulted in 0.5 % CD144 + cells; CHIR 9902 2 µM added to the priming medium resulted in 14.6 % CD144+ cells; 2 µM Compound 21 (GP21R7) added to the priming medium resulted in 40.2 % CD144 + cells and 2 µM Compound 21 (CP21R7) added to the priming medium plus addition of Forskolin 5 µM to the induciton medium Day 4 induction medium + VEGF-A 50 ng/ml (lower panel, left) and Day 4 induction medium resulted in 54.6 % CD 144 + cells

Figures 4 shows that the differentiated cells from hESC and hiPSCs are able to form capillary-like tube formation (hESC derived: 90 % / hiPSC derived: 80%).

Figures 5 shows that the differentiated cells from hESC and hiPSCs are positive for CD31, vWF, CD 144, VEGFR-2 and thus qualify as endothelial cells.

Figure 6 shows the comparison of a prior art method (Tatsumi et al) and the method of the invention. The method of Tatsumi et al employs the BIO inhibitor at different concentrations as supplement to the N2B27. Although Tatsumi et al. describes that the pluripotent stem cells were differentiated to endothelial cells with about 20% efficiency (determined by endothelial cells expressing the surface marker CD144), we were not able to reproduce such results. Moreover, a strong cytotoxicity of the BIO inhibitor already at concentrations of 1 µM was observed.

The sequential supplementation of the priming step (ii) with 25 ng/ml BMP4 together with 1 µM CP and the induction step (iii) with 200 ng/ml VEGF together with 2 µM Forskolin rapidly induced CD 144 expression in up to 85% of cells on day six. Overall robustness and reproducibility of the differentiation protocol was observed in different human ESC and iPSC lines (Figure 8).

The derivation of highly functional endothelial cells for drug discovery programs and clinical applications requires large numbers of quality-controlled cell populations. To address this point, magnetic-activated cell sorting (MACS) was used to separate cells into CD144⁺ and CD 144- cell fractions. Using separation settings designed for highly-stringent isolation of positive-labeled cells, purities greater than 95% of CD144⁺ cells were achieved. (Figure 20).

### 3. Cryopreservation protocol:

1x10⁶ cells are suspended in 1 ml cryopreservation medium (90% FBS and 10% DMSO) and transferred into cryo vial. Cryo vials are placed in Nalgene Cryo 1°C Freezing Container to achieve a -1°C/min cooling rate when stored at -80°C. Freezing container is stored at -80°C for 24 hours. For long term storage cells are subsequently transferred into liquid Nitrogen.

Figure 7 shows that the 8x10⁵ (80% of the total frozen cell number) of Cryopreserved PSC-derived ECs are viable after thawing. Large majority of the thawed cells attached over night to fibronectin-coated dishes and grow adherently on the next day. Very few floating (dead) cells are observed. Purity and identity of crypopreserved PSC-ECs was maintained in terms of endothelial specific marker expression (CD31, CD34, CD144, KDR).

**Table 1: Reproducibility of the method for differentiation of pluripotent stem cells into endothelial cells.**

| **Experiment Date** | **hPSCs Number Day0** | **ECs after MACS Sorting Day5** | **Purity CD144+ cells** |
|---|---|---|---|
| 02.03.2011 | 3x10⁶ cells | 2.4x10⁶ cells | 97% |
| 18.04.2011 | 3x10⁶ cells | 2.6x10⁶ cells | 85% |
| 27.05.2011 | 3x10⁶ cells | 1.9x10⁶ cells | 97% |

### 4. Endothelial induction by pharmacological inhibition of GSK3β

The potential of the most widely used commercial GSK3β inhibitors and CP21R7 to activate β-catenin-mediated TCF/LEF transcription in human PA-1 reporter cells (DeAlmeida et al., 2007) was evaluated. Compounds used are shown in Figure 18. A dose-response assay revealed that all of the most potent GSK3 inhibitors namely CP21R7 (CP), 6-bromoindirubin-3'-oxime (BIO), CB36155 and CHIR-99021 (CHIR) exhibited a steep activation curve suggesting co-operative binding (Figure 19). Unmodified PA-1 cells are used to measure cellular viability via ATP levels and GLI-luciferase responsive reporter PA-1 cells as counter screen to monitor changes in general transcriptional activity. Overall the analyzed Gsk3β inhibitors showed an insignificant rise in general transcriptional activity (data not shown). A three-fold increase in GLI-mediated luciferase activity was observed with the treatment of BIO, which was highly toxic at concentration of 10 µM (data not shown). CB361549 and CB361556 (MeBIO) as well as Forskolin (negative control) did not induce luciferase expression. The concentration dependent administration of CP revealed a bell-shaped curve with highest luciferase activity at 3 µM. The maximum of a 566 fold increased luciferase activity was accompanied with only a modest gain of cell proliferation and minor increases of general transcriptional activity. The concentration dependent administration of previously described GSK3β inhibitor SB216763 resulted in negligible β-catenin mediated TCF/LEF luciferase expression. On the outcome of the β-Catenin reporter assay, three GSk3β inhibitors namely BIO, CHIR and CP were exploited for their potential to drive vascular lineage commitment of human PSCs. In addition to the listed inhibitors we include SB216763 (SB) due to its structure similarity to CP (see Figure 18). Pluripotent stem cells were (i) plated as single-cells on mTeSR1 medium supplemented with Y-27632 a Rho-kinase inhibitor (Watanabe et al., 2007),; (ii) incubated in priming medium (N2B27 medium supplemented with a GSK3β inhibitor) and (iii) incubated in induction medium (StemPro SFM 34 medium supplemented with Vascular Endothelial Growth Factor (VEGF; Sumi et al., 2008)) as outlined above under 1 (i) and (ii).

### The following GSK3 inhibitors were used:

CHIR99021 GSK3-Inhibitor: Catalogue Number: 361559 from Merck Millipore
SB216763 GSK3-Inhibitor: Catalogue Number: 361566 from Merck Millipore
BIO GSK3-Inhibitor: Catalogue Number: 3 361550 from Merck Millipore
CP21R7: 3-(3-Amino-phenyl)-4-(1-methyl-1H-indol-3-yl)-pyrrole-2,5-dione (also referred to as "compound 21" herein; see e.g. L. Gong et al; Bioorganic& Medicinal Chemistry Letters 20 (2010), 1693-1696).

Proliferation into vascular bed cells was detected on Day 5. Vascular identity is confirmed by the expression of vascular endothelial-cadherin (VE-Cadherin). The capability to induce vascular bed cells was determined by VE-Cadherin (CD144) expression on day 5 (Figure 19). Flow cytometric analysis identified CP as the far most potent GSK3β inhibitor. The concentration dependent treatment resulted in a bell-shaped curve with steep slopes confirming previous findings of the TCF/LEF-luciferase reporter assay. Remarkably, up to 35% of cells expressed CD 144 at an optimal concentration of 1 µM from day five on. The small compound BIO appeared to be toxic on single cells (data not shown). CHIR, which has a divergent chemical structure than CP induced CD 144 expression also in a parabolic curve reaching an optimum at 6 µM. In accordance to the TCF/LEF-luciferase reporter assay no CD 144 expression was observed after administering SB to the differentiation platform (Figure 19). Although, SB and CP share the same chemical backbone, divergent moieties may influence the chemical properties, e.g. cellular permeability or solubility. In carrier treated cells no VE-CADHERIN expression was detected (Figure 19).

### 5. Differentiation dynamics

WNT/β-Catenin signaling is fundamental to induce primitive streak formation (Tam and Loebel, 2007). For this reason, the temporal appearance of meseodermal progenitor cells was analysed. Genome-wide gene expression analysis revealed that 24 hours upon combined CP and BMP4 treatment, pluripotency markers such as *NANOG*, *UTF1* and *SOX2* are downregulated (data not shown). Upregulation of representative markers: *MIXL1, T*/*BRACHYURY, FGF4* and *EOMES* indicated that cell lineage decision is directed into mesoderm within the same time frame. In the course of the differentiation we observed no elevated gene expression of markers aligned with neuroectoderm and endoderm (data not shown). The gene *SOX17,* a marker for the onset of endoderm formation, was highly expressed from day 5 on. This finding is in accordance with the *in vivo* expression pattern of *SOX17* in the developing and adult vasculature (Engert et al., 2009). No lineage commitment into trophectoderm or visceral endoderm was observed, as relevant genes such as *ESX1 and SOX7* were not detected (data not shown).

Immunostainings of SOX 17, OCT4, T-BRACHYURY, PECAM-1 and VE-CADHERIN support the transcriptome data (Quantification shown in Figure 9). The protein expression of the pan-mesodermal marker T-BRACHYURY confirms an intermediate PS-like cell stage. The treatment with CP and BMP4 induced T-BRACHYURY expression quickly after one day. The vast majority of cells reached a T-BRACHYURY peak expression on day three. On day four the expression diminished and disappeared, thereby mimicking its specific embryonic expression pattern (Tam and Loebel, 2007). Loss of pluripotency was monitored by the time-dependent decline of OCT4 expression. Pharmacological inhibition of GSK3ß with CP21 alone differentiated PSCs into a salt and pepper staining pattern of SOX17⁺ cells at day three; whereas combined application of CP and BMP4 prevented the appearance of endodermal SOX17⁺ cells (Figure 9). Induction of endothelial cell identity was determined by the co-expression of platelet-endothelial cell adhesion molecule-1 (PECAM1) and VE-CADHERIN. Endothelial marker expression was detected upon administration of VEGF on day 4 (Figure 9). This finding was completed by the transcriptome analysis of endothelial markers *CD34, PECAM1*, *CDH5* and *vWF*, which were upregulated upon VEGF more than 100 fold within the first 24 hours, respectively (data not shown). Our differentiation system enables high throughput derivation of endothelial cells within 5 days through a transient emergence of primitive streak cells.

### 6. Protocol for differentiation of PSCs into VSMC

When using the differentiation protocol described above, two distinct cell populations appeared after transfer of unsorted cells into StemPro medium supplemented with VEGF. Cell types differed by morphology as well as by marker expression. Cobblestone-like cells with tight cell-cell contacts expressed the endothelial-specific marker VE-Cadherin. Flattened and spindle-like shaped cells stained positive for Smooth Muscle Actin (αSMA), indicating a vascular smooth muscle cell (VSMC) identity. In flow cytometric analysis cells separated into a major fraction of CD31⁺/CD140⁻ cells and minor fraction of CD31⁻/CD140b⁺ cells (Figure 10).

Replacement of the induction medium of step (ii) by an RPMI medium with 0.5 µM IWR (inhibitor of Wnt response compound 1) resulted in an inverted distribution of CD31⁺/CD 140band CD31⁻/CD140b⁺ cells.

The adapted differentiation system induced PDGFRβ expression in up to 90% of the cells. Immunostainings of SM22a and αSMA confirmed the VSMC identity of CD31⁻/CD140b⁺ cells (Figure 10).

With this new protocol it is now possible to predominantly induce either endothelial cells ore vascular smooth muscle cells. A summary of the different protocols is shown in Figure 11.

Further characterization of MACS-purified CD144⁺ cells endorsed an endothelial phenotype. CD144⁺ cells have an uniform cobblestone-like morphology and present an endothelial-specific expression pattern; positive for CD31, CD34, CD105, CD146, KDR, PECAM1, VE-CADHERIN, vWF (von-Willebrand factor), ZO1 (zona occludens 1) and negative for the hematopoietic lineage markers CD43 and CD45 (Figure 12). The stem cell-derived ECs (SC-derived ECs) still have a proliferative capability at least up to passage 5 and stain positive for ID1 and c-Kit indicating the precursor state of the cells (data not shown). CD144⁺-purified cells were cultured until confluence and thereafter cryopreserved. Banks of Stem cell-derived endothelial cells are amenable to high throughput approaches required for drug discovery campaigns.

### 7. Functional characterization of stem cell-derived vascular bed cells

**Ac-LDL-uptake:** Cells were incubated in medium containing 2.5 µg/mL Alexa Fluor 594 acetylated low-density lipoprotein (AlexaFluor594-Ac-LDL) from Molecular Probes/Invitrogen for 4 h at 37°C. After incubation, cells were washed and fixed with 4% PFA for 10 min. Incorporation of Alexaflor594-Ac-LDL was visualized with a fluorescent microscope.

**Pericyte association assay:** Human Brain Vascular Pericytes (HBVPs) were purchased from ScienCell Research Laboratories. HBVPs were grown in Pericyte Medium (ScienCell Research Laboratories). For the functional tube formation and association assays the SC-ECs and the HBVPs were grown using the in vitro angiogenesis a kit from AMS biotechnology Immediately before the sowing for functional assays the HBVPs were stained red and the SC-ECs were stained green by using Cell Tracker dyes from Invitrogen according to the manufactures instructions. For the functional assays equal numbers (2x104/cm2) of both cell types were grown in EC expansion medium (StemPro-34 with VEGF).

**Cell-based ELISA protocol:** 20 000 Stem Cell-derived endothelial cells/well were seeded onto 96-well plates and cultured for 2 days in EGM-2 medium (Lonza) until confluence. Media was changed with EGM-2 medium supplemented with 1 nM TNFα (R&D Systems). After an incubation of 24 hours SC-derived ECs were briefly PBS-washed and PFA-fixed Primary antibodies for human anti-ICAM and anti-E-Selectin were purchased from R&D Systems. An anti-mouseIgG/biotinylated (Amersham) as secondary antibody and a Streptavidin/Peroxidase complex (Amersham) were used. Luminescence was quantified in a microplate reader.

**Leukocyte adhesion assay:** 2x10⁴ Stem Cell-derived endothelial cells/well were seeded onto 96-well plates and cultured for 2 days in EGM-2 medium (Lonza) until confluence. Media was changed with EGM-2 medium supplemented with 1 nM TNFα (R&D Systems). SC-derived ECs were TNFα-stimulated for 4 hours. Thereafter 1.5x10⁵ Calcein-labeled HL60 cells were added to each 96well. After an incubation of one hour medium was removed and Sc-ECs were PBS-washed. Lyse the plate with 1% NP-40 detergent solution for 15 minutes. Supernatants were transferred into optiplates and fluorescence measured in a microplate reader.

HL60 Leukocytes (Collins et al., 1977; Nature, 270, 347) are grown in RPMI-1640 medium (Invitrogen) with 10%FCS.

In order to define the endothelial phenotype more profoundly, the angiogenic potential, the ability to uptake DiI-acetylated low-density lipoprotein (DiI-ac-LDL) was assessed and the dynamic modulation of the barrier function was monitored. For tube formation assays SC-derived ECs were seeded onto a Matrigel matrix.

SC-derived ECs rapidly formed network-like structures with a similar pattern as reference endothelial cells (data not shown).

Treatment of angiogenesis inhibitors such as Sulforaphane and anti-VEGF monoclonal antibodies perturbed tube formation and reduced the number of nodes as well as the length of tubes (data not shown). Interestingly, cellular interaction of SC-derived ECs with mural self-arranged themselves in highly organized tube-like structures.

Human brain vascular pericytes (HBVPs) primarily associated to SC-derived ECs contributing to tubular structures. HBVPs cells appeared to envelope SC-derive endothelial cells by winding their cell body around the tubes (Figure 13). HBVPs alone are not capable of forming tubular structures on the Matrigel matrix. Either the presence of SC-derived ECs or the addition of platelet-derived growth factor (PDGF) is required to instruct tube formation of vascular mural cells (data not shown). A similar self-arrangement was observed when SC-derived VSMCs were used as mural cell source (data not shown). Mural cell properties of SC-derived VSMC were also assured in migration/wound healing assays in which SC-derived VCMCs extending into the wound edge as free single cells (Gottlieb and Spector, 1981) (data not shown).

The dynamic monitoring of monolayer formation and thrombin induced permeability followed by barrier recovery was measured with the xCeLLigence RT-CA system (Atienza et al., 2006; Solly et al., 2004). The system provides a label free real-time monitoring of the density-dependent proliferation and viability of the SC-derived ECs. The interaction of the ECs with the microelectrodes leads to a change in impedance that is proportional to the cell number and morphology as well as the tightness of cell attachment (Atienza et al., 2006). Our data show that after a *log* growth phase, the SC-derived ECs reach a plateau, which they can obtain for several days without major fluctuations (data not shown). After the initial attachment, spreading and proliferation the SC-derived ECs achieved a tight monolayer by interacting with each other in a dynamic fashion through VE-Cadherins and amongst other tight/adherens junction proteins (data not shown). The observed rapid and reversible effect of SC-derived ECs to the vasoactive agent thrombin is accompanied by cell rounding and inter-endothelial gap formation (data not shown). Thrombin stimulation of endothelial cells causes transient alterations of the VE-Cadherins and the associated catenins (Marie-Josèphe et al., 1996) resulting in a reversible disruption of the permeability properties (data not shown). Aside from forming tube-like structures and a tight monolayer, SC-derived ECs are take up DiI-Ac-LDL from the growth medium (Figure 14). Overall SC-derived ECs exhibit endothelial-like functionality in the performed *in vitro* assays.

### 8. SC-derived ECs functionally respond to proinflammatory cytokines

In response to proinflammatory stimuli ECs express cellular adhesion molecules (CAMs) including intracellular adhesion molecule-1 (ICAM1) and E-SELECTIN. Activated ECs enable the capturing of leukocytes and tethering them to the locus of inflammation via the CAMs (Rao et al., 2007; Galkina et al., 2007). Vascular inflammation plays a central role in the initiation and progression of atherosclerotic plaque formation (Losis, 2000).

To determine whether the differentiation system generates *bona fide* ECs, we challenged the SC-derived ECs with proinflammatory cytokines and analyzed for an activated expression of effectors of immune cell migration. Immunofluorescence analysis clearly showed an increased expression of ICAM1 upon TNF-α treatment (Figure 15). We further confirmed by flow cytometry the presence of ICAM1 and E-SELECTIN upon TNF-α as well as IL-1β stimulation (Figure 16). HLA-ABC expression served as control to analyze a general und cell type unspecific response to proinflammatory cytokines. We also performed a cell-based ELISA to measure the activation of ICAM1 and E-SELECTIN in a dose response to TNF-α. SC-derived ECs present ICAM1 and E-SELECTIN in expression levels comparable to primary human umbilical vein endothelial cells (HUVECs; data not shown). We next used SC-derived ECs in co-culture with HL60 leukocytes, to investigate whether the adhesion molecules of activated SC-derived ECs exhibit a biological function. It turned out that the adhesion of HL60 leukocytes was significantly enhanced when SC-derived ECs were stimulated with TNF-α (Figure 16). In order to assess whether the adhesion is specifically mediated, we co-treated SC-ECs with an anti-ICAM monoclonal antibody. Indeed, inhibition of ICAM1 could prevent the adhesion of HL60 in a concentration dependent mode (Figure 16). We performed static adhesion assays with HUVECs to confirm HL60 leukocyte adhesion as endothelial specific property, which can be inhibited by anti-ICAM1 antibody treatment (data not shown).

**Scratch assay:** Stem Cell-derived vascular smooth muscle cells (SC-derived VSMC) were grown until confluence. A scratch of VSMC monolayer was removed using a 200µl pipette tip. Images of the same area were taken after scratching to determine scratch closure.

## Claims

1. A method for differentiating pluripotent stem cells into vascular bed cells, said method comprising the steps of:
a) providing a monolayer of pluripotent stem cells in a pluripotency medium
b) incubating said cells in a priming medium supplemented with a small molecule that activates the Beta-catenin and/or Wnt signaling and/or Hedgehog (HH) signaling, wherein the small molecule is 3-(3-Amino-phenyl)-4-(1-methyl-1 H-indol-3-yl)-pyrrole-2,5-dione,
c) inducing the differentiation by incubating said primed cells in an induction medium.

2. The method of claim 1 for differentiating pluripotent stem cells into endothelial cells.

3. The method of claim 1 for differentiating pluripotent stem cells into vascular smooth muscle cells.

4. The method of any of claims 1 to 3, wherein step a) additionally comprises incubating said cells in the pluripotency medium for 18 hours to 30 hours.

5. The method of any of claims 1 to 4, wherein step b) additionally comprises incubating said cells in the priming medium for 2 to 4 days.

6. The method of any of claims 1 to 5, wherein step c) additionally comprises incubating said cells in the induction medium for 18 hours to 48 hours.

7. The method of any of claims 1 to 6, wherein said pluripotency medium of step a) is a serum-free medium supplemented with an inhibitor of the Rho-associated coiled-coil forming protein serine/threonine kinase family of protein kinases (ROCK kinase inhibitor).

8. The method of claim 7, wherein said ROCK kinase inhibitor is selected from the group of 1-(5-Isoquinolinesulfonyl) homopiperazine), N-Benzyl-2-(pyrimidin-4-ylamino) thiazole-4-carboxamide) and (+)-(R)-trans-4-(1-aminoethyl)-N-(4-pyridyl) cyclo-hexanecarboxamide dihydrochloride).

9. The method of any of claims 1 to 8, wherein said priming medium of step b) is a serum free medium supplemented with insulin, transferrin and progesterone.

10. The method of any of claims 1 to 9, wherein said priming medium of step b) additionally comprises recombinant bone morphogenic protein-4 (BMP4).

11. The method of any of claims 1 to 10, wherein said induction medium is a serum-free medium supplemented with VEGF-A (Vascular endothelial growth factor) or placenta like growth factor 1 (PLGF-1) and a small molecule adenylate cyclase activator.

12. The method of claim 11, wherein said small molecule adenylate activators is selected from the group comprising Forskolin ((3R)-(6aalphaH)Dodecahydro-6beta, 10alpha,10balpha-trihydroxy-3beta,4abeta,7,7,10abeta-pentamethyl-1-oxo-3-vinyl-1H-naphtho[2,1-b]pyran-5beta-yl acetate), 8-Bromo-cAMP (8-Bromoadenosine-3',5'-cyclic monophosphate) and Adrenomedullin.

13. The method of any of claims 1 to 10, wherein said induction medium is a serum replacement medium or a serum-free medium supplemented with activators of TGFbeta signaling and PDGF signaling.

14. The method of any of claims 1 to 13, wherein said pluripotent stem cell is an induced pluripotent stem cell.

15. The method of claim 14, wherein said induced pluripotent stem cell is a human cell.

16. The method of claims 14 or 15, wherein said induced pluripotent stem cell is obtained from a subject suffering from a disease associated with vascular complications.

17. The method of any of claims 1 to 16, additionally comprising the step of d) incubating the product of step c) under conditions suitable for proliferation of the endothelial cells or vascular smooth muscle cells.

18. The method of any one of claims 1 to 17, further comprising generating a biobank of endothelial cells or vascular smooth muscle cells.

## Patentansprüche

1. Verfahren zur Differenzierung von pluripotenten Stammzellen in Gefäßzellen (vascular bed cells), wobei das Verfahren die Schritte umfasst:
a) Bereitstellen einer Monoschicht von pluripotenten Stammzellen in einem Medium für die Kultur pluripotenter Stammzellen (pluripotency medium),
b) Inkubieren der Zellen in einem Priming-Medium, das mit einer niedermolekularen Verbindung (small molecule) supplementiert ist, die die Beta-Catenin- und/oder Wnt-Signalgebung und/oder die Hedgehog (HH)-Signalgebung aktiviert, wobei die niedermolekulare Verbindung 3-(3-Amino-phenyl)-4-(1-methyl-1 H-indol-3-yl)pyrrol-2,5-dion ist,
c) Induzieren der Differenzierung durch Inkubieren der geprimten Zellen in einem Induktionsmedium.

2. Verfahren nach Anspruch 1 zur Differenzierung pluripotenter Stammzellen in Endothelzellen.

3. Verfahren nach Anspruch 1 zur Differenzierung pluripotenter Stammzellen in vaskuläre glatte Muskelzellen.

4. Verfahren nach einem der Ansprüche 1 bis 3, wobei Schritt a) zusätzlich das Inkubieren der Zellen in dem Medium für die Kultur pluripotenter Stammzellen über einen Zeitraum von 18 Stunden bis 30 Stunden umfasst.

5. Verfahren nach einem der Ansprüche 1 bis 4, wobei Schritt b) zusätzlich das Inkubieren der Zellen in dem Priming-Medium über einen Zeitraum von 2 bis 4 Tagen umfasst.

6. Verfahren nach einem der Ansprüche 1 bis 5, wobei Schritt c) zusätzlich das Inkubieren der Zellen in dem Induktionsmedium über einen Zeitraum von 18 Stunden bis 48 Stunden umfasst.

7. Verfahren nach einem der Ansprüche 1 bis 6, wobei das Medium für die Kultur pluripotenter Stammzellen von Schritt a) ein serumfreies Medium ist, das mit einem Inhibitor der Rho-assoziierten Coiled-Coil bildenden Serin/Threonin-Protein-Kinase-Familie der Proteinkinasen (Rho-associated coiled-coil forming protein serine/threonine kinase family of protein kinases; ROCK-Kinase-Inhibitor) supplementiert ist.

8. Verfahren nach Anspruch 7, wobei der ROCK-Kinase-Inhibitor ausgewählt ist aus der Gruppe bestehend aus 1-(5-Isochinolinsulfonyl)-homopiperazin, N-Benzyl-2-(pyrimidin-4-ylamino)thiazol-4-carboxamid) und (+)-(R)-trans-4-(1-aminoethyl)-N-(4-pyridyl)-cyclo-hexancarboxamid-dihydrochlorid.

9. Verfahren nach einem der Ansprüche 1 bis 8, wobei das Priming-Mdium von Schritt b) ein serumfreies Medium ist, das mit Insulin, Transferrin und Progesteron supplementiert ist.

10. Verfahren nach einem der Ansprüche 1 bis 9, wobei das Priming-Medium von Schritt b) zusätzlich rekombinantes knochenmorphogenetisches Protein 4 (bone morphogenic protein-4; BMP4) umfasst.

11. Verfahren nach einem der Ansprüche 1 bis 10, wobei das Induktionsmedium eine serumfreies Medium ist, das mit VEGF-A (vascular endothelial growth factor) oder Plazenta-ähnlicher Wachstumsfaktor 1 (placenta like growth factor 1; PLGF-1) und einem niedermolekularen Aktivator der Adenylatcyclase supplementiert ist.

12. Verfahren nach Anspruch 11, wobei der niedermolekulare Aktivator der Adenylatcyclase ausgewählt ist aus der Gruppe bestehend aus Forskolin ((3R)-(6aalphaH)Dodecahydro-6beta,10alpha,10balpha-trihydroxy-3beta,4abeta,7,7,10abeta-pentamethyl-1-oxo-3-vinyl-1H-naphto[2,1-b]pyran-5beta-yl-acetat, 8-Bromo-cAMP (8-Bromadenosin-3',5'-cyclisches Monophosphat) und Adrenomedullin.

13. Verfahren nach einem der Ansprüche 1 bis 10, wobei das Induktionsmedium ein Serumersatzmedium oder ein serumfreies Medium ist, das mit Aktivatoren der TGFbeta-Signalgebung und der PDGF-Signalgebung supplementiert ist.

14. Verfahren nach einem der Ansprüche 1 bis 13, wobei die pluripotente Stammzelle eine induzierte pluripotente Stammzelle ist.

15. Verfahren nach Anspruch 14, wobei die induzierte pluripotente Stammzelle eine humane Zelle ist.

16. Verfahren nach Anspruch 14 oder 15, wobei die induzierte pluripotente Stammzelle von einem Individuum erhalten wird, das an einer Erkrankung leidet, die mit Gefäßkomplikationen assoziiert ist.

17. Verfahren nach einem der Ansprüche 1 bis 16, das zusätzlich den Schritt d) des Inkubierens des Produkts aus Schritt c) unter Bedingungen, die für die Proliferation der Endothelzellen oder vaskulären glatten Muskelzellen geeignet sind, umfasst.

18. Verfahren nach einem der Ansprüche 1 bis 17, das des Weiteren das Erzeugen einer Biobank von Endothelzellen oder vaskulären glatten Muskelzellen umfasst.

## Revendications

1. Procédé pour différencier des cellules souches pluripotentes en cellules de lit vasculaire, ledit procédé comprenant les étapes de :
a) fourniture d'une couche monocellulaire de cellules souches pluripotentes dans un milieu de pluripotence,
b) mise en incubation desdites cellules dans un milieu d'amorçage additionné d'une petite molécule qui active la bêta-caténine et/ou la transmission de signal Wnt et/ou la transmission de signal Hedgehog (HH), ladite petite molécule étant la 3-(3-aminophényl)-4-(1-méthyl-1H-indole-3-yl)-pyrrole-2,5-dione,
c) induction de la différenciation par mise en incubation desdites cellules amorcée dans un milieu d'induction.

2. Procédé suivant la revendication 1 pour différencier des cellules souches pluripotentes en cellules endothéliales.

3. Procédé suivant la revendication 1 pour différencier des cellules souches pluripotentes en cellules de muscle lisse vasculaire.

4. Procédé suivant l'une quelconque des revendications 1 à 3, dans lequel l'étape a) comprend en outre la mise en incubation desdites cellules dans le milieu de pluripotence pendant 18 heures à 30 heures.

5. Procédé suivant l'une quelconque des revendications 1 à 4, dans lequel l'étape b) comprend en outre la mise en incubation desdites cellules dans le milieu d'amorçage pendant 2 à 4 jours.

6. Procédé suivant l'une quelconque des revendications 1 à 5, dans lequel l'étape c) comprend en outre la mise en incubation desdites cellules dans le milieu d'induction pendant 18 heures à 48 heures.

7. Procédé suivant l'une quelconque des revendications 1 à 6, dans lequel ledit milieu de pluripotence de l'étape a) est un milieu sans sérum additionné d'un inhibiteur de la famille de protéines-kinases du type protéine-sérine/ thréonine-kinase de formation de spires associées à Rho (inhibiteur de kinase ROCK).

8. Procédé suivant la revendication 7, dans lequel ledit inhibiteur de kinase ROCK est choisi dans le groupe de la 1-(5-isoquinoléinesulfonyl)homopipérazine), du N-benzyl-2-(pyrimidine-4-ylamino)thiazole-4-carboxamide) et du dichlorhydrate de (+)-(R)-trans-4-(1-aminoéthyl)-N-(4-pyridyl)-cyclohexanecarboxamide.

9. Procédé suivant l'une quelconque des revendications 1 à 8, dans lequel ledit milieu d'amorçage de l'étape b) est un milieu sans sérum additionné d'insuline, de transférine et de progestérone.

10. Procédé suivant l'une quelconque des revendications 1 à 9, dans lequel ledit milieu d'amorçage de l'étape b) comprend en outre la protéine morphogène osseuse recombinante 4 (BMP4).

11. Procédé suivant l'une quelconque des revendications 1 à 10, dans lequel ledit milieu d'induction est un milieu sans sérum additionné de VEGF-A (facteur de croissance endothélial vasculaire) ou de facteur de croissance de type placentaire 1 (PLGF-1) et d'un activateur d'adénylate-cyclase en petites molécules.

12. Procédé suivant la revendication 11, dans lequel ledit activateur d'adénylate en petites molécules est choisi dans le groupe comprenant la forskoline (acétate de (3R)-(6aalphaH)dodécahydro-6bêta,10alpha,10balpha-trihydroxy-3bêta,4abêta,7,7,10abêta-pentamethyl-1-oxo-3-vinyl-1H-naphto-[2,1-b]pyrane-5bêta-yle), le 8-bromo-AMPc (8-bromo-adénosine-3',5'-monophosphate cyclique) et l'adrénomédulline.

13. Procédé suivant l'une quelconque des revendications 1 à 10, dans lequel ledit milieu d'induction est un milieu de substitution de sérum ou un milieu sans sérum additionné d'activateurs de transmission de signal de TGF bêta et de transmission de signal de PDGF.

14. Procédé suivant l'une quelconque des revendications 1 à 13, dans lequel ladite cellule souche pluripotente est une cellule souche pluripotente induite.

15. Procédé suivant la revendication 14, dans lequel ladite cellule souche pluripotente induite est une cellule humaine.

16. Procédé suivant la revendication 14 ou 15, dans lequel ladite cellule souche pluripotente induite est obtenue à partir d'un sujet souffrant d'une maladie associée à des complications vasculaires.

17. Procédé suivant l'une quelconque des revendications 1 à 16, comprenant en outre l'étape d) de mise en incubation du produit de l'étape c) dans des conditions convenables pour la prolifération des cellules endothéliales ou des cellules de muscle lisse vasculaire.

18. Procédé suivant l'une quelconque des revendications 1 à 17, comprenant en outre la génération d'une biobanque de cellules endothéliales ou de cellules de muscle lisse vasculaire.
